# EUROPEAN PATENT APPLICATION

(11) **EP 4 737 568 A1**
(43) Date of publication of application: **06.05.2026**
(21) Application number: 24832553.2
(22) Date of filing: 24.06.2024
(51) Int. Cl.: C12N 15/10, C12N 9/22, C12N 15/113, C12Q 1/6897

(54) **NOVEL CRISPR/CAS13 SYSTEM AND USE THEREOF**

(30) Priority: 30.06.2023 KR 20230084898
(71) Applicant: Nsage Corp., Incheon 21999 (KR)
(72) Inventor: LEE, Bong Hee, Incheon 22009 (KR); BAYARSAIKHAN, Delger, Incheon 21990 (KR); BAYARSAIKHAN, Govigerel, Incheon 21990 (KR); GASIUNAS, Giedrius, 06285 Vilnius (LT); STITILYTE, Migle, 08336 Vilnius (LT); KOO, Okjae, Seoul 03626 (KR); KANG, Hyun A, Incheon 21982 (KR); LEE, Jae Suk, Seoul 07281 (KR)
(74) Representative: dompatent
(86) International application number: PCT/KR2024/095888
(87) International publication number: WO 2025/005777

(57) **Abstract**

The present specification discloses a novel CRISPR/Cas 13 system. The novel CRISPR/Cas13 system was identified from a public database. The novel /Cas 13 system has both target-specific nucleic acid cleavage activity and collateral cleavage activity. The inventors of the present specification have specified a novel CRISPR/Cas 13 system from a known database, and have demonstrated the function thereof through experimentation, thereby completing the invention.

## Description

### Technical Field

The present invention relates to a CRISPR/Cas 13 system, which belongs to a Class 2, Type VI CRISPR/Cas system.

### Background Art

### CRISPR/Cas 13 system

### Outline of CRISPR/Cas 13 system

This specification discloses a novel CRISPR/Cas 13 system, which is a type of CRISPR/Cas system. The CRISPR/Cas 13 system includes an effector protein (Cas 13 protein) and a guide RNA. The CRISPR/Cas 13 system recognizes sequences complementary to a spacer sequence of the guide RNA, and a single-stranded RNA that has Protospacer Flanking Sequence (PFS), and cleaves them. In some CRISPR/Cas 13 systems, when the single-stranded RNA is cleaved, a collateral cleavage activity is triggered, and then the single-stranded RNA neighboring the system is randomly cleaved.

The following provides a more detailed explanation of a structure and a function of the CRISPR/Cas 13 system.

### Classification of CRISPR/Cas 13 system

A CRISPR/Cas 13 system belongs to Class 2, Type VI CRISPR/Cas systems. The CRISPR/Cas 13 system is further divided into several subtypes, including Cas 13a, Cas 13b, Cas 13c, Cas 13d, Cas 13x, and Cas 13y. These subtypes are also referred to as IV-a, IV-b, IV-c, IV-d, IV-x, and IVy, respectively. The CRISPR/Cas 13 systems within each subtype exhibit slight differences in a size of a Cas 13 protein and a structure of a guide RNA, etc.

### Composition and function of CRISPR/Cas 13 system

A CRISPR/Cas 13 system includes a Cas 13 protein and a guide RNA. These Cas 13 protein and guide RNA function as a complex. This protein-RNA complex can bind to a target nucleic acid (recognize the target nucleic acid) and cleave it. Additionally, a certain protein-RNA complex has a collateral cleavage activity, wherein the complex randomly cleaves nucleic acids neighboring the complex after cleaving the target nucleic acid. This Cas 13 protein-guide RNA complex is also referred to as a Ribonucleoprotein (RNP). The Cas 13 protein can recognize a Protospacer Flanking Sequence (PFS) and cleave nucleic acids. The guide RNA can direct the Cas 13 protein-guide RNA complex to the target nucleic acid by binding to the target nucleic acid that has the target sequence.

The CRISPR/Cas 13 system is known to function by targeting single-stranded RNA as a target nucleic acid.

### Structure of Cas 13 protein

The Cas 13 protein can be broadly divided into a RECognition (REC) lobe and a NUClease (NUC) lobe. The NUC lobe contains two Higher Eukaryotes and Prokaryotes Nucleotide-binding (HEPN) domains. The REC lobe plays a crucial part when the Cas 13 protein recognizes a guide RNA and forms a complex. The NUC lobe cleaves nucleic acids. A structure of the Cas 13 protein has been reported by various researchers. For instance, it is explained in detail by Xue et al.(Engineering CRISPR/Cas 13 System against RNA Viruses: From Diagnostics to Therapeutics. Bioengineering (Basel), July 2022; 9(7): 291).

### Structure of guide RNA

A guide RNA of a CRISPR/Cas 13 system, unlike that of the CRISPR/Cas 9 system, is composed of single RNA molecule, referred to as a crRNA. The guide RNA includes a direct repeat and a spacer.

A connection structure between the direct repeat and the spacer varies depending on a subtype of the CRISPR/Cas 13 system. For instance, in guide RNAs of Cas 13a and Cas 13d subtypes, the direct repeat and the spacer are connected sequentially from a 5'-end to a 3'-end direction. In contrast, in a guide RNA of a Cas 13b subtype, a spacer and a direct repeat are connected sequentially from a 5'-end to a 3'-end direction.

### Target-specific nucleic acid cleavage activity of CRISPR/Cas 13 system, and Protospacer Flanking Sequence (PFS)

The CRISPR/Cas 13 system can cleave nucleic acids with target specificity. Two conditions have to be met for target-specific nucleic acid cleavage activity to exhibit. First, a nucleic acid has to contain a sequence of bases having certain length, which can be recognized by a Cas 13 protein. Second, there has to be a sequence that can bind complementarily to a spacer included in a guide RNA neighboring the sequence of bases having certain length. When 1) the Cas 13 protein recognizes the sequence of bases having certain length, and 2) the spacer binds complementarily to a region neighboring the sequence of bases having certain length, the CRISPR/Cas 13 system cleaves nucleic acids. Here, the sequence of bases having certain length recognized by the Cas 13 protein is referred to as a Protospacer Flanking Sequence (PFS), which has a concept corresponding to the Protospacer Adjacent Motif (PAM) of CRISPR/Cas 9 systems.

The PFS is determined by a type of the Cas 13 protein. Knowing the PFS of the Cas 13 protein allows for designing CRISPR/Cas 13 systems that target nucleic acids neighboring the PFS by using the knowledge.

Among Cas 13 subtypes, the CRISPR/Cas 13d system is known as not requiring the PFS. The CRISPR/Cas 13d system can cleave target nucleic acids without additional conditions (irrespective of a presence of the PFS sequence), as long as the spacer of the guide RNA binds complementarily to a target region.

### Collateral cleavage activity of CRISPR/Cas 13 system

Some CRISPR/Cas 13 systems have a collateral cleavage function in addition to a target-specific nucleic acid cleavage activity. When the CRISPR/Cas 13 system recognizes a target nucleic acid, its collateral cleavage function is activated. The CRISPR/Cas 13 system with its collateral cleavage function activated randomly cleaves single-stranded nucleic acids neighboring the CRISPR/Cas 13 system. This collateral cleavage function can be applied to detect the presence of target nucleic acids in a sample using the CRISPR/Cas 13 system.

### Disclosure

### Technical Problem

This specification is intended to disclose a novel CRISPR/Cas 13 system that has not been reported previously. This specification is intended to disclose each component of the novel CRISPR/Cas 13 system. This specification is intended to elucidate functions of the novel CRISPR/Cas 13 system. This specification is intended to elucidate the possible use of the novel CRISPR/Cas 13 system.

### Technical Solution

The inventors of this specification identified components of the novel CRISPR/Cas 13 system from microbial genetic sequence information disclosed in public databases. Specifically, the present inventors identified a Cas 13 protein of SEQ ID NO: 1 and a guide RNA containing a direct repeat sequence of SEQ ID NO: 2. Based on specific information, the inventors produced the novel CRISPR/Cas 13 system. Furthermore, the inventors confirmed that the novel CRISPR/Cas 13 system can cleave nucleic acids with target specificity through experiments. Additionally, it was confirmed that the novel CRISPR/Cas13 system belongs to a Cas 13d subtype and can function without limitations to a Protospacer Flanking Sequence (PFS). Furthermore, the inventors also confirmed that through experiments, the novel CRISPR/Cas 13 system has a collateral cleavage function.

The inventors found that the novel CRISPR/Cas 13 system can be used for cleaving non-target nucleic acids or detecting a target nucleic acid. The inventors specifically proposed and demonstrated methods of cleaving non-target nucleic acids and detecting the target nucleic acid.

### Advantageous Effects

A novel CRISPR/Cas 13 system of this specification can cleave single-stranded nucleic acids with target specificity. Since the novel CRISPR/Cas 13 system has a collateral cleavage activity, it can recognize the target nucleic acid and randomly cleave nucleic acids neighboring the CRISPR/Cas 13 system. This characteristic of the novel CRISPR/Cas 13 system can be utilized to detect the target nucleic acid.

### Description of Drawings

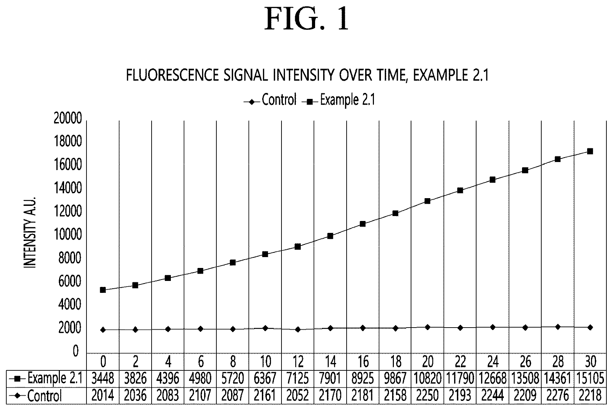

Figure 1 shows results of measuring a fluorescence signal intensity over time according to Experimental Example 2.1. A vertical axis of a chart represents a relative fluorescence intensity, and a horizontal axis represents a reaction time in minutes. "Example 2.1" indicates results for Example 2.1 set forth in Table 3, while "Control" represents results obtained by adding only Cas 13 protein.

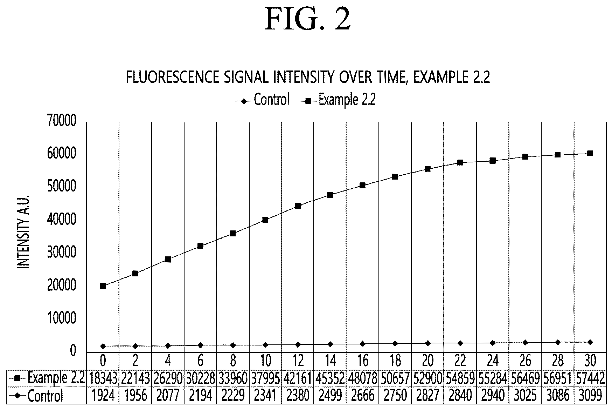

Figure 2 shows results of measuring a fluorescence signal intensity over time according to Experimental Example 2.1. A vertical axis of a chart represents a relative fluorescence intensity, while a horizontal axis represents a reaction time in minutes. "Example 2.2" indicates results for Example 2.2 set forth in Table 3, and "Control" represents results obtained by adding only Cas 13 protein.

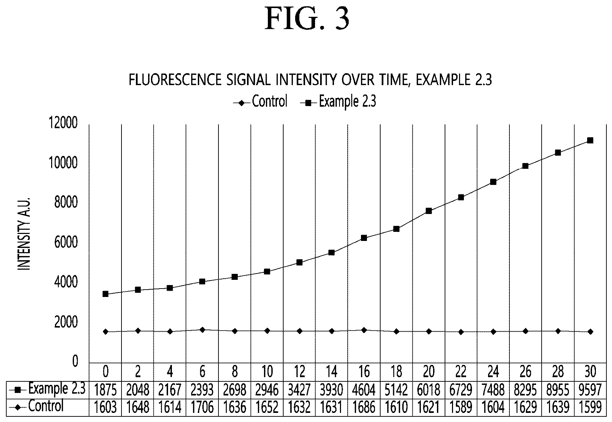

Figure 3 shows results of measuring a fluorescence signal intensity over time according to Experimental Example 2.1. A vertical axis of a chart represents a relative fluorescence intensity, while a horizontal axis represents a reaction time in minutes. "Example 2.3" indicates results for Example 2.3 set forth in Table 3, and "Control" represents results obtained by adding only Cas 13 protein.

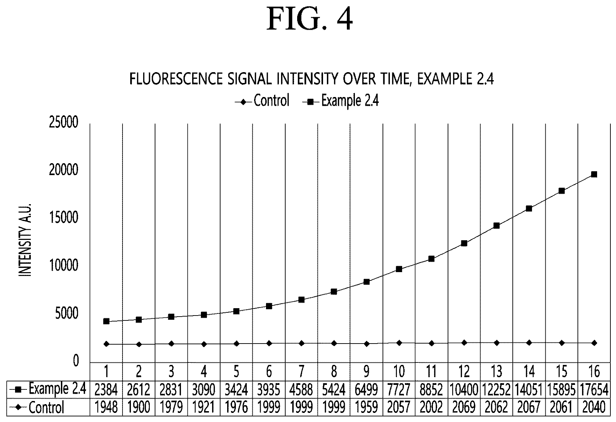

Figure 4 shows results of measuring a fluorescence signal intensity over time according to Experimental Example 2.1. A vertical axis of a chart represents a relative fluorescence intensity, while a horizontal axis represents a reaction time in minutes. "Example 2.4" indicates results for Example 2.4 set forth in Table 3, and "Control" represents results obtained by adding only Cas 13 protein.

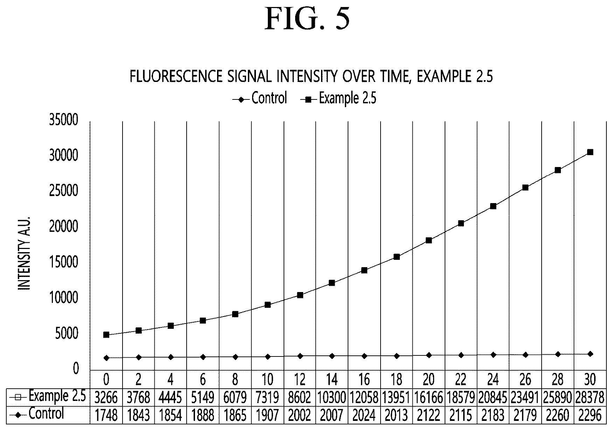

Figure 5 shows results of measuring a fluorescence signal intensity over time according to Experimental Example 2.1. A vertical axis of a chart represents a relative fluorescence intensity, while a horizontal axis represents a reaction time in minutes. "Example 2.5" indicates results for Example 2.5 set forth in Table 3, and "Control" represents results obtained by adding only Cas 13 protein.

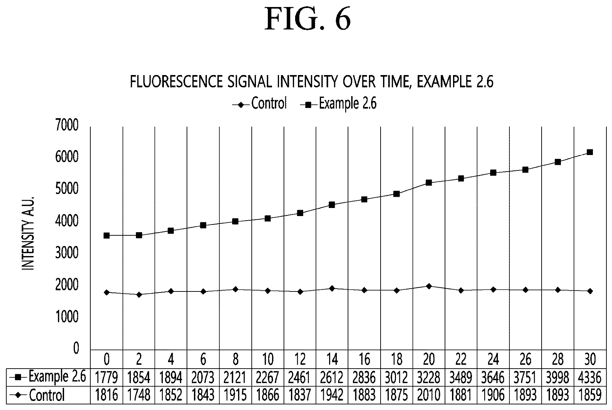

Figure 6 shows results of measuring a fluorescence signal intensity over time according to Experimental Example 2.1. A vertical axis of a chart represents a relative fluorescence intensity, while a horizontal axis represents a reaction time in minutes. "Example 2.6" indicates results for Example 2.6 set forth in Table 3, and "Control" represents results obtained by adding only Cas 13 protein.

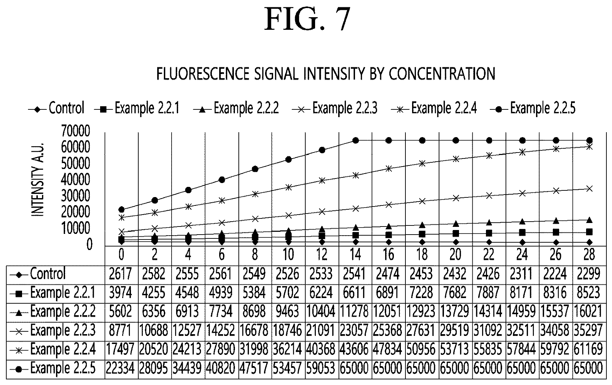

Figure 7 shows results of measuring a fluorescence signal intensity at various concentrations according to Experimental Example 2.2. A vertical axis of a chart represents a relative fluorescence intensity, while a horizontal axis represents a reaction time in minutes. "Example 2.2.1" through "Example 2.2.5" correspond to Examples indicated by labels disclosed in Table 4 respectively, while "Control" represents results obtained by adding only Cas 13 protein.

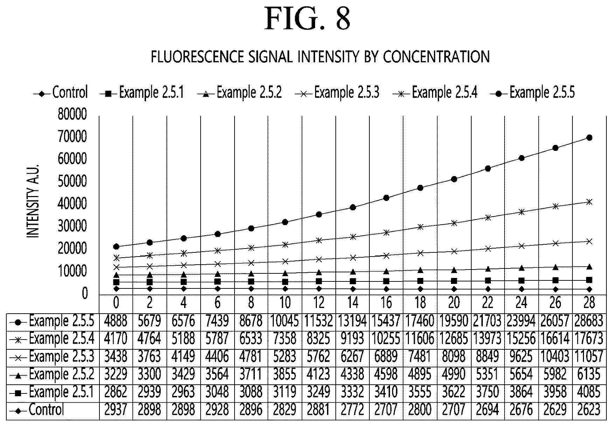

Figure 8 shows results of measuring a fluorescence signal intensity at various concentrations according to Experimental Example 2.2. A vertical axis of a chart represents a relative fluorescence intensity, while a horizontal axis represents a reaction time in minutes. "Example 2.5.1" through "Example 2.5.5" correspond to Examples indicated by labels disclosed in Table 4 respectively, while "Control" represents results obtained by adding only Cas 13 protein.

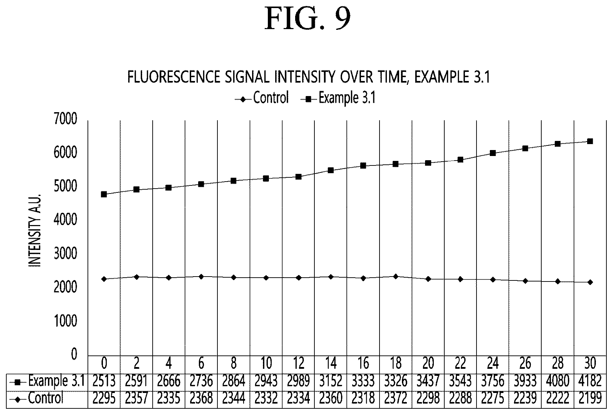

Figure 9 shows results of measuring a fluorescence signal intensity over time according to Experimental Example 3.1. A vertical axis of a chart represents a relative fluorescence intensity, and a horizontal axis represents a reaction time in minutes. "Example 3.1" indicates results for Example 3.1 set forth in Table 4, while "Control" represents results obtained by adding only Cas 13 protein.

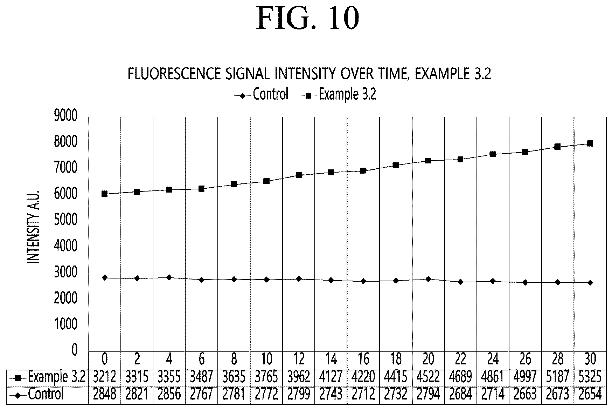

Figure 10 shows results of measuring a fluorescence signal intensity over time according to Experimental Example 3.1. A vertical axis of a chart represents a relative fluorescence intensity, and a horizontal axis represents a reaction time in minutes. "Example 3.2" indicates results for Example 3.2 set forth in Table 4, while "Control" represents results obtained by adding only Cas 13 protein.

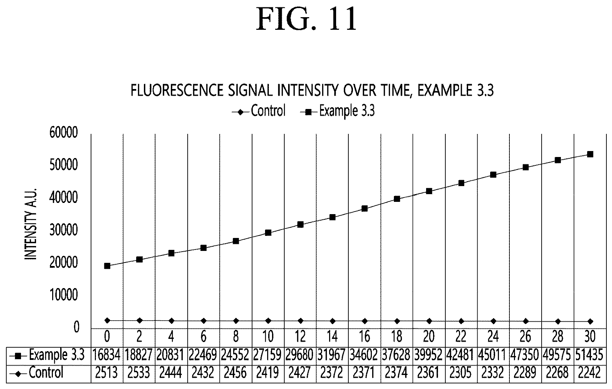

Figure 11 shows results of measuring a fluorescence signal intensity over time according to Experimental Example 3.1. A vertical axis of a chart represents a relative fluorescence intensity, and a horizontal axis represents a reaction time in minutes. "Example 3.3" indicates results for Example 3.3 set forth in Table 4, while "Control" represents results obtained by adding only Cas 13 protein.

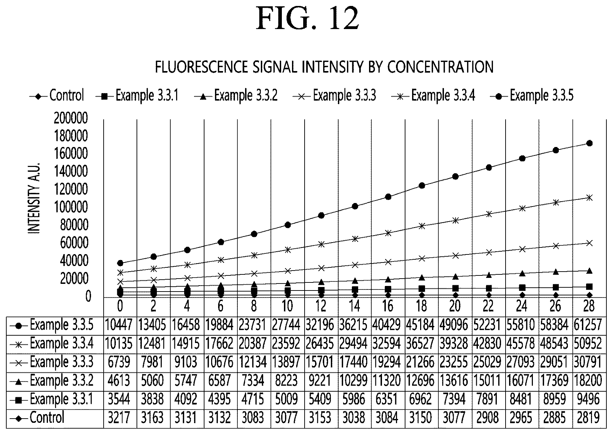

Figure 12 shows results of measuring a fluorescence signal intensity at various concentrations according to Experimental Example 3.2. A vertical axis of a chart represents a relative fluorescence intensity, while a horizontal axis represents a reaction time in minutes. "Example 3.3.1" through "Example 3.3.5" correspond to Examples indicated by labels set forth in Table 6 respectively, while "Control" represents results obtained by adding only Cas 13 protein.

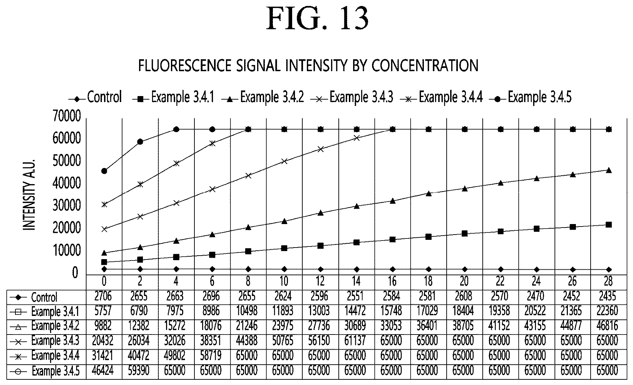

Figure 13 shows results of measuring a fluorescence signal intensity at various concentrations according to Experimental Example 3.2. A vertical axis of a chart represents a relative fluorescence intensity, while a horizontal axis represents a reaction time in minutes. "Example 3.4.1" through "Example 3.4.5" correspond to Examples indicated by labels set forth in Table 6 respectively, while "Control" represents results obtained by adding only Cas 13 protein.

### Best Mode

Below, this specification will disclose the best mode for carrying out the present invention exemplarily. This includes some embodiments of the invention disclosed in this specification, but does not include all embodiments. The embodiments described in this paragraph are merely examples, and the embodiments described in this paragraph should not be understood as the "best mode for carrying out the invention". A person of ordinary skill in the art would be able to conceive many variations and more preferable embodiments based on the examples described in this paragraph, and such variations and embodiments should also be considered as parts of the best mode for carrying out the invention.

The specification discloses a novel CRISPR/Cas 13 composition including: a Cas 13 protein comprising a nucleic acid sequence of SEQ ID NO: 1, or a nucleic acid encoding the Cas 13 protein; and a programmable guide RNA, or a nucleic acid encoding the programmable guide RNA, wherein the programmable guide RNA has a structure where a direct repeat and a guide domain are linked from a 5' end to a 3' end direction, wherein the direct repeat interacts with the Cas 13 protein to form a complex, wherein the direct repeat comprises a RNA sequence of SEQ ID NO: 2, wherein the guide domain can bind complementarily to a predetermined target nucleic acid.

In one embodiment, the nucleic acid encoding the Cas 13 protein may be DNA or mRNA, and the nucleic acid encoding the programmable guide RNA may be DNA.

In one embodiment, the novel CRISPR/Cas 13 composition may comprise the Cas 13 protein and the programmable guide RNA, and the Cas 13 protein and the programmable guide RNA may form a protein-RNA complex.

In one embodiment, the novel CRISPR/Cas 13 composition may comprise the nucleic acid encoding the Cas 13 protein and the nucleic acid encoding the programmable guide RNA.

In one embodiment, the nucleic acid encoding the Cas 13 protein and the nucleic acid encoding the programmable guide RNA may be included in single vector molecule.

In one embodiment, the nucleic acid encoding the Cas 13 protein and the nucleic acid encoding the programmable guide RNA may be each included in two or more vector molecules.

In one embodiment, the vector may be a viral vector, a non-viral vector, or a proper combination thereof.

The specification discloses a method of cleaving non-target RNAs using a novel CRISPR/Cas 13 system, comprising: mixing a novel CRISPR/Cas 13 composition with a sample, wherein the sample comprises a target RNA and the non-target RNA, wherein a guide domain of a programmable guide RNA of the novel CRISPR/Cas 13 composition is capable of binding complementarily to the target RNA.

The specification discloses a method of detecting a target RNA using a novel CRISPR/Cas 13 system, comprising: (a) mixing a novel CRISPR/Cas 13 composition, the sample, and a detecting agent, wherein a guide domain of a programmable guide RNA of the novel CRISPR/Cas 13 composition is capable of binding complementarily to the target RNA, wherein the detecting agent comprises a reporter, and the reporter comprises a probe nucleic acid, wherein when the probe nucleic acid is cleaved, the reporter generates a detectable signal, wherein when the sample comprises the target RNA, a protein-RNA complex of the novel CRISPR/Cas 13 composition binds complementarily to the target RNA, thereby activating an collateral cleavage function of the protein-RNA complex, leading to a cleavage of the probe nucleic acid; and (b) measuring the detectable signal in the mixture, wherein information on the target RNA can be obtained by measuring the detectable signal.

In one embodiment, the (b) may be a process of measuring: whether the detectable signal is generated; an intensity of the generated signal; a change in signal intensity over time; or any combination thereof.

In one embodiment, information about the target RNA may comprise whether the target RNA is present, how much of the target RNA is included in a sample, or both.

In one embodiment, the detectable signal may be a fluorescence signal.

The specification discloses a method of providing information on diagnosing COVID-19, comprising: (a) mixing a novel CRISPR/Cas 13 complex, a specimen, and a detecting agent, wherein the specimen is collected from a patient suspected of having COVID-19 infection, wherein the novel CRISPR/Cas 13 complex comprises: a Cas 13 protein of SEQ ID NO: 1; and a guide RNA; wherein the guide RNA has a structure where a direct repeat of SEQ ID NO: 2 and a guide domain are linked sequentially from a 5' end to a 3' end direction, wherein the guide domain comprises a RNA sequence selected from a group consisting of SEQ ID NOS: 37 to 51, and SEQ ID NOS: 52 to 56, wherein the detecting agent comprises a reporter, and the reporter comprises a probe nucleic acid, wherein when the probe nucleic acid is cleaved, the reporter generates a fluorescence signal; (b) measuring the fluorescence signal in the mixture; and (c) obtaining information on whether a patient who provides a specimen is infected with COVID-19, by analyzing the fluorescence signal.

In one embodiment, the (b) may be a process of measuring whether a fluorescence signal is generated; an intensity of the fluorescence signal generated; a change in the fluorescence signal intensity over time; or any combination thereof.

### Mode for Invention

Below, with reference to attached drawings, the present disclosure is described in more detail through specific embodiments and examples. It should be noted that the attached drawings include some embodiments of the invention, but do not include all embodiments. The invention disclosed in this specification may be implemented in various ways and is not limited to the specific embodiments described herein. These embodiments should be considered as being provided to meet the legal requirements applicable to the specification. A person skilled in the art to which the present invention pertains would be able to conceive many modifications and other embodiments based on the invention disclosed in this specification. Therefore, the invention disclosed in this specification is not limited to the specific embodiments described herein, and variations and other embodiments thereof should be understood to fall within the scope of the claims.

### Definition

### About

The term "about" as used in this specification refers to a specified amount, level, value, number, frequency, percentage, dimension, size, quantity, weight, or length that varies by about 30, 25, 20, 15, 10, 9, 8, 7, 6, 5, 4, 3, 2, 1%, or 0% of a reference amount, level, value, number, frequency, percentage, dimension, size, quantity, weight, or length.

### Description of amino acid sequence

Unless otherwise stated, the amino acid sequence in this specification is described using either a one-letter code or a three-letter code for amino acids, in the direction from a N-terminus to a C-terminus. For example, when represented as VSKN, it refers to a peptide consisting of valine (Val), serine (Ser), lysine (Lys), and asparagine (Asn) sequentially connected from a N-terminus to a C-terminus. As an another example, when represented as Thr-Leu-Lys, it refers to a peptide consisting of threonine (Thr), leucine (Leu), and lysine (Lys) sequentially connected from a N-terminus to a C-terminus. For amino acids that cannot be represented by the one-letter code, other characters are used, along with additional explanations provided.

The notation methods for each amino acid are as follows: Alanine (Ala, A); Arginine (Arg, R); Asparagine (Asn, N); Aspartic acid (Asp, D); Cysteine (Cys, C); Glutamic acid (Glu, E); Glutamine (Gln, Q); Glycine (Gly, G); Histidine (His, H); Isoleucine (Ile, I); Leucine (Leu, L); Lysine (Lys, K); Methionine (Met, M); Phenylalanine (Phe, F); Proline (Pro, P); Serine (Ser, S); Threonine (Thr, T); Tryptophan (Trp, W); Tyrosine (Tyr, Y); and Valine (Val, V).

### Description of nucleic acid sequence

Symbols A, T, C, G, and U used in this specification are to be interpreted as meanings understood by those skilled in the art. Depending on the context and the technology, the symbols can be appropriately interpreted as bases, nucleosides, or nucleotides on DNA or RNA. For example, when the symbols A, T, C, G, or U refer to bases, they can be interpreted as adenine(A), thymine(T), cytosine(C), guanine(G), or uracil(U), respectively. When the symbols A, T, C, G, or U refer to nucleosides, they can be interpreted as adenosine (A), thymidine (T), cytidine(C), guanosine(G), or uridine(U), respectively. When the symbols A, T, C, G, or U refer to nucleotides within a sequence, they should be interpreted as nucleotides containing the respective nucleosides.

### Discovery of novel CRISPR/Cas 13 systems from public database

The inventors of this specification discovered that the novel CRISPR/Cas 13 system from public databases. The inventors first identified a Cas 13 protein and a guide RNA from microbial genetic sequence information in public databases. Then, the inventors synthesized the identified Cas 13 protein and the guide RNA, and produced a CRISPR/Cas 13 complex. The inventors confirmed that the produced CRISPR/Cas 13 complex exhibits a target-specific nucleic acid cleavage activity and a collateral cleavage function, thereby completing the present invention.

Specifically, the present inventors utilized genetic information published by National Center for Biotechnology Information (NCBI). By decoding metagenome sequencing data of a bovine gut disclosed by NCBI, the inventors obtained genomic information on whether each microorganism is present in bovine gut fluid. Based on a known sequence information on Cas 13 proteins, the inventors identified genes encoding CRISPR/Cas 13 systems among microbial genes found in camel stomach. From these genes, the inventors identified an amino acid sequence of the Cas 13 protein and the guide RNA sequence. The inventors selected candidates that exhibit particularly excellent collateral cleavage function from the CRISPR/Cas 13 system candidates obtained in this manner.

### Novel CRISPR/Cas 13 system

### Outline of novel CRISPR/Cas 13 system

The specification discloses the novel CRISPR/Cas 13 system. The novel CRISPR/Cas 13 system was discovered from public databases. The novel CRISPR/Cas 13 system can cleave a target nucleic acid. The novel CRISPR/Cas 13 system can also cleave any nucleic acids neighboring the CRISPR/Cas 13 system after recognizing the target nucleic acid. In other words, the novel CRISPR/Cas 13 system has both a target-specific nucleic acid cleavage activity and a collateral cleavage activity. The novel CRISPR/Cas 13 system belongs to a Cas 13d subtype. The novel CRISPR/Cas 13 system can recognize and cleave the target nucleic acid without limitation to a PFS. The inventors of this specification identified the novel CRISPR/Cas 13 system from public databases and demonstrated its function through experiments, thereby completing the present invention.

### Novel Cas 13 protein - amino acid sequence

The novel CRISPR/Cas 13 system includes the novel Cas 13 protein. The novel Cas 13 protein is an ortholog of the Cas 13 protein. Although the novel Cas 13 protein was identified from public databases, its function or application had not been previously known. The novel Cas 13 protein can form a complex with a programmable guide RNA which will be described below. The novel Cas 13 protein includes an amino acid sequence of SEQ ID NO: 1 or a similar amino acid sequence thereto.

### Novel Cas 13 protein - PFS sequence

The Cas 13 protein can exhibit the aforementioned target-specific nucleic acid cleavage function and/or the collateral cleavage function when (1) the target nucleic acid includes a specific sequence, and (2) a sequence adjacent to the specific sequence can complementarily bind to a guide RNA. In this case, this specific sequence is referred to as a Protospacer Flanking Sequence (PFS). The PFS is a sequence adjacent to a 5'-end, a 3'-end, or both ends of the sequence (target sequence) that can complementarily bind to the guide RNA.

### Novel Cas 13 protein - PFS sequence, in the case of the Cas13d subtype

The CRISPR/Cas 13 system belonging to the Cas 13d subtype are known not to require the PFS. The CRISPR/Cas 13d system functions as long as a target sequence can complementarily bind to a guide RNA. Experiments have confirmed that the novel CRISPR/Cas 13 system disclosed in this specification belongs to the Cas 13d subtype. Therefore, the novel CRISPR/Cas 13 system can recognize the target nucleic acid and perform its functions only with a programmable guide RNA, without limitation to the PFS.

### Programmable guide RNA - structure of guide RNA

The novel CRISPR/Cas 13 system includes a programmable guide RNA. The programmable guide RNA includes a direct repeat and a guide domain. The programmable guide RNA is connected to the direct repeat and the guide domain sequentially in the direction from a 5'-end to a 3'-end. In other words, the programmable guide RNA includes the following structure:
5' - (direct repeat) - (guide domain) - 3',
wherein an additional domain can be further included between the direct repeat and the guide domain.

Each domain will be described below in more detail.

### Programmable guide RNA - direct repeat

The direct repeat is a part which forms a stem-loop structure. The direct repeat makes the programmable guide RNA interact with the novel Cas 13 protein to form a complex. A nucleic acid sequence of the direct repeat may vary depending on the Cas 13 protein.

### Programmable guide RNA - guide domain

The guide domain corresponds to a spacer of the guide RNA described in the <<CRISPR/Cas 13 system>>. The guide domain is artificially designed based on a predetermined target sequence. How the guide domain is designed determines which nucleic acids will be able to be targeted by the CRISPR/Cas 13 system.

### Function of novel CRISPR/Cas 13 system - target RNA cleavage activity

The novel CRISPR/Cas 13 system can cleave a target RNA. When the novel CRISPR/Cas 13 complex binds to the target RNA, the complex can cleave it. A programmable guide RNA of the novel CRISPR/Cas 13 system is designed based on the target RNA. The programmable guide RNA is designed to complementarily bind to the target RNA.

### Function of novel CRISPR/Cas 13 system - collateral cleavage activity

The novel CRISPR/Cas 13 system has a collateral cleavage activity. When the novel CRISPR/Cas 13 system binds to a target RNA, the collateral cleavage function is activated. When the collateral cleavage function is activated, the novel CRISPR/Cas 13 system randomly cleaves single-stranded RNA neighboring the CRISPR/Cas 13 system. The nucleic acid sequence of the single-stranded RNA cleaved by the collateral cleavage activity is unrelated to a sequence of the target RNA. Regardless of the nucleic acid sequences, it is cleaved by the novel CRISPR/Cas 13 system with activated collateral cleavage function.

### Novel CRISPR/Cas 13 complex

The novel CRISPR/Cas 13 system may refer to a complex of the Cas 13 protein and the programmable guide RNA. This complex can be referred to as a protein-RNA complex or a ribonucleoprotein (RNP).

### Expression vector for each component of novel CRISPR/Cas 13 system

The novel CRISPR/Cas 13 system may refer to a vector capable of expressing the Cas 13 protein and the programmable guide RNA. The expression vector is not limited to its composition or form, as long as it can express each component of the novel CRISPR/Cas 13 system. The expression vector may include a nucleic acid encoding the Cas 13 protein and a nucleic acid encoding the programmable guide RNA, as well as other components such as promoter. The expression vector can be DNA, mRNA, or an appropriate combination thereof.

### Novel CRISPR/Cas 13 composition

The novel CRISPR/Cas 13 system may refer to a novel CRISPR/Cas 13 composition.

The novel CRISPR/Cas 13 composition includes the followings: the novel Cas 13 protein or a nucleic acid encoding the Cas 13 protein; and the programmable guide RNA or a nucleic acid encoding the programmable guide RNA.

### Use of novel CRISPR/Cas 13 system

The novel CRISPR/Cas 13 system can bind to a predetermined target RNA based on a design of a programmable guide RNA (target RNA recognition function). The novel CRISPR/Cas 13 system can cleave the predetermined target RNA (target-specific nucleic acid cleavage activity). The novel CRISPR/Cas 13 system can randomly cleave single-stranded RNA neighboring the CRISPR/Cas 13 system regardless of its sequence after cleaving the predetermined target RNA (collateral cleavage activity). When these characteristics are used, the CRISPR/Cas 13 system can be utilized to cleave non-target RNAs with any nucleic acid sequences. Additionally, the CRISPR/Cas 13 system can be utilized to detect a target RNA in a sample. The use of this system will be described below in more detail.

### Method of cleaving non-target nucleic acids utilizing novel CRISPR/Cas 13 system

### Outline of method of cleaving non-target nucleic acids

The specification discloses a method of cleaving non-target nucleic acids using the novel CRISPR/Cas 13 system. The method of cleaving non-target nucleic acids utilizes a characteristic of the novel CRISPR/Cas 13 system, wherein when the novel CRISPR/Cas 13 system recognizes a target nucleic acid, the collateral cleavage function of the system is activated, thereby cleaving any nucleic acids. The method of cleaving non-target nucleic acids includes a process of contacting the target nucleic acid and the non-target nucleic acids with the novel CRISPR/Cas 13 system. The novel CRISPR/Cas 13 system is described in the paragraph <<Novel CRISPR/Cas 13 System>>. A guide domain of the guide RNA of the CRISPR/Cas 13 system is designed to target the target nucleic acid.

Below, the above method will be described in more detail.

### Novel CRISPR/Cas 13 system

To cleave the non-target nucleic acids, the novel CRISPR/Cas 13 system provided in this specification is used. The novel CRISPR/Cas 13 system is described in the <<Novel CRISPR/Cas 13 System>>. Depending on the purpose, a manner in which the CRISPR/Cas 13 system is used may vary. For example, a novel CRISPR/Cas 13 complex, expression vector for each component of the novel CRISPR/Cas 13 system, a novel CRISPR/Cas 13 composition, or appropriate combinations thereof may be utilized.

### Target nucleic acid

In the method of cleaving non-target nucleic acids, the target nucleic acid refers to a nucleic acid that can be recognized by the novel CRISPR/Cas 13 system. Specifically, the target nucleic acid can hybridize with a guide domain of a programmable guide RNA of the novel CRISPR/Cas 13 system. In other words, the target nucleic acid includes a nucleic acid sequence that is complementary to the guide domain.

### Non-target nucleic acids

The non-target nucleic acids refer to single-stranded nucleic acids that can be cleaved by a Cas 13 protein of the novel CRISPR/Cas 13 system. Unlike the target nucleic acid, the non-target nucleic acids have no limitation to their sequences or length.

### Order of contacting target nucleic acid and non-target nucleic acids

A method of cleaving the non-target nucleic acids utilizes a collateral cleavage function of the novel CRISPR/Cas 13 system. The collateral cleavage function is activated when the novel CRISPR/Cas 13 system recognizes a target nucleic acid. Therefore, it is preferable to perform the method of cleaving the non-target nucleic acids in the order that the novel CRISPR/Cas 13 system contacts a target nucleic acid, followed by the non-target nucleic acids. However, even if the system contacts the non-target nucleic acids first, as long as it is sufficiently adjacent to the novel CRISPR/Cas 13 system, the non-target nucleic acids can also be cleaved when the target nucleic acid contacts the system later. In conclusion, the order in which the novel CRISPR/Cas 13 system contacts the target nucleic acid and the non-target nucleic acids are not critically important when performing the method of cleaving the non-target nucleic acids. The order in which the novel CRISPR/Cas 13 system contacts the target nucleic acid and the non-target nucleic acids can vary as necessary when performing the method of cleaving the non-target nucleic acids. In some cases, the target nucleic acid and the non-target nucleic acids can simultaneously contact the novel CRISPR/Cas 13 system.

### Method of detecting target nucleic acid using novel CRISPR/Cas 13 system

### Outline of method of detecting target nucleic acid

This specification discloses a method of detecting a target nucleic acid using a novel CRISPR/Cas 13 system. The method of detecting the target nucleic acid utilizes a collateral cleavage activity of the novel CRISPR/Cas 13 system. The method of detecting the target nucleic acid includes a process of mixing the novel CRISPR/Cas 13 system with a sample and a detecting agent; and a process of measuring whether a predetermined signal is generated in the mixture. Here, the sample is a specimen that may contain a target nucleic acid. The detecting agent includes a reporter that generates a specific signal under specific conditions. The reporter contains a probe nucleic acid, and when the probe nucleic acid is cleaved, the reporter generates a detectable signal (e.g., fluorescence). When the sample contains a target nucleic acid, the novel CRISPR/Cas 13 system recognizes the target nucleic acid. When the novel CRISPR/Cas 13 system recognizes the target nucleic acid, the collateral cleavage function is activated, which randomly cleaves nucleic acids neighboring the CRISPR/Cas 13 system. As a result, when the probe nucleic acid of the reporter is cleaved, a detectable signal is generated. When the detectable signal is measured and analyzed, it is possible to determine whether the sample contains the target nucleic acid and how much of the target nucleic acid is present.

Below, each component and process used in this method will be described in detail.

### Sample

A method of detecting a target nucleic acid using the novel CRISPR/Cas 13 system is conducted with a sample. By performing this method, it can be determined whether the sample contains the target nucleic acid and, how much of the target nucleic acid is present. In other words, the sample is the subject of detecting the target nucleic acid. The sample can take various forms as necessary. For example, the sample may include body fluid from humans or non-human animals. As an another example, the sample may be a specimen derived from the pre-treatment of body fluid from humans or non-human animals.

### Novel CRISPR/Cas 13 system

The novel CRISPR/Cas 13 system is used to determine whether the sample contains a target nucleic acid and how much of the target nucleic acid is present. The novel CRISPR/Cas 13 system is described as in <<Novel CRISPR/Cas 13 System>>. The novel CRISPR/Cas 13 system can be used in the form of a novel CRISPR/Cas 13 complex, expression vectors for each component of the novel CRISPR/Cas 13 system, a novel CRISPR/Cas 13 composition, or an appropriate combination thereof.

### Detecting agent

When the sample contains a target nucleic acid, a detecting agent is used to generate a detectable signal. The detecting agent includes a reporter, which contains a probe nucleic acid. The reporter emits a detectable signal when the probe nucleic acid is cleaved. The detectable signal can be, for example, a fluorescence signal. In case that the sample contains a target nucleic acid, the novel CRISPR/Cas 13 system can bind to the target nucleic acid. When the novel CRISPR/Cas 13 system binds to the target nucleic acid, a collateral cleavage function is activated. When the collateral cleavage function is activated, nucleic acids neighboring the novel CRISPR/Cas 13 system are randomly cleaved. Then, when a probe nucleic acid of the reporter is cleaved, the reporter generates a detectable signal. In conclusion, when a sample, a novel CRISPR/Cas 13 system, and a detecting agent are mixed together, a presence of a target nucleic acid in the sample will provide a detectable signal generated by the reporter.

The detecting agent may further include additional substances, which can be variously selected as necessary. For example, the additional substances may be substances that promote a collateral cleavage activity of a novel CRISPR/Cas 13 system. As an another example, the additional substances may be substances that amplify a detectable signal generated by the reporter.

### Detectable signal

The detectable signal refers to a signal that can be detected during a target nucleic acid detection method. This is determined by a type of reporter included in the detecting agent. As long as the goal of the method can be achieved, the identity of the detectable signal is not important. The signal generated by the reporter can vary. For instance, the signal may be fluorescence at a specific wavelength. By measuring: whether the detectable signal is generated, how strong the signal is, and how a signal intensity changes, etc., information on the target nucleic acid can be obtained. Such information on the target nucleic acid may include, for instance whether a target nucleic acid is present in a sample, and how much of the target nucleic acids are present in the sample, etc.

### Process of mixing sample, CRISPR/Cas 13 system, and detecting agent

As described above, a detectable signal is generated when a target nucleic acid, the novel CRISPR/Cas 13 system, and a reporter contact each other. Therefore, to detect the target nucleic acid, a sample, the novel CRISPR/Cas 13 system, and the detecting agent must all be brought together in one place. This process can be described in various ways. For example, the sample, the novel CRISPR/Cas 13 system, and the detecting agent can be brought together in one place by mixing, combining, reacting, contacting each other, or appropriately combining thereof. This process should be conducted under conditions where this series of steps - where the novel CRISPR/Cas 13 system recognizes the target nucleic acid, activates the collateral cleavage function to cleave the probe nucleic acid of the reporter, generates the detectable signal from the reporter, and measures the signal - is not hindered. As long as these conditions are satisfied, the process is not limited by a method, environment, or location of performance. For simplicity, hereafter, this process will be referred to as "mixing".

### Order of mixing sample, CRISPR/Cas 13 system and detecting agent

In the method of detecting a target nucleic acid, the order of mixing the sample, the novel CRISPR/Cas 13 system, and the detecting agent is not particularly significant. The sample, the novel CRISPR/Cas 13 system, and the detecting agent can be mixed simultaneously or in any order. According to principle of the method of detecting the target nucleic acid, it may be considered preferable to mix the sample and the novel CRISPR/Cas 13 system, followed by the detecting agent. However, as long as mixing is performed in an appropriate period, the series of reactions described above can occur sufficiently. The order of mixing the sample, the novel CRISPR/Cas 13 system, and the detecting agent does not significantly impact an effect of the method of detecting the target nucleic acid.

### Process of measuring detectable signal

The method of detecting a target nucleic acid includes a process of measuring a detectable signal. As previously described, when the target nucleic acid is present in a sample, a reporter of a detecting agent emits the detectable signal through a series of reactions. The method of measuring the detectable signal is determined based on an identity of the signal emitted and the pattern in which the signal is generated. This method is not particularly limited and known methods may also be utilized.

### Possible embodiments of present invention_

### Cas 13 protein

### Example 1. Novel Cas 13d protein

A Cas 13 protein of VI-D subtype (also referred to as Cas 13d protein)

### Example 2. Novel Cas 13 protein

In example 1, the Cas 13 protein includes an amino acid sequence of

VSKNDNIKSKAKALGLKSTFQVGDEVVMTSFGKGNKAIVEKIVRGTDVQSVPTEPNF
SAEIEGKKFDLVGRAHIQTKSDNPQYSKKRTGDDMIGAKAALEKRFFGGTFDDNIHI
QLAYNVLDIEKILSVHINNIVYTLNNIRRKDNAEDDDFIGYMSTRNDYDTFIEPRKH
NISEDAAKSIDKSRASFEEYLQPPVKDCLHYFGNTFFAPREIEKTYTDNRGFERKKK
VTVNSLIDEKEIYYIFALLGGLRQFCTHDNKSGRNWLYSLEENGINSDAKAVLDKYY
NSAVARIDESFVDNASKTNFKLIFNAMDVSDEALQDNIAKAFYKFTVCKSFKNMGFS
IKKLREQLLELPEYEGLKDKHYDSVRSKLYQIMDFVIYLSFKDEKFKKDNEEKINNI
VNELRATLNEEDKGRVYASYAERMKSELKPAIARLKSDIDKIKDSRVKEFELDASVK
YRLSKVVESVRLKDRATYFTKLIYLTTLFLDGKEINDLLTTLIHQFENIASFIDVMN
DRGIDCRFSDGYKLFESSKQIAFELRNVNSFARMTRTSKDDENATHMMYIDAAEILG
TDYTEEQIEEHLNLEKKRMIPGTKKADMNFRNFIINNVIKSSRFNYLVRYSNPKKIR

ALADNEGVIRFVLGELPDAQIDRYTLLCGFNPDADRQEKTDKLAKAITGLRFNDFEN
VKQGANTEGESQESIDKAQKQGLISLYLTVLYLLTKNLVYVNSRYFLAFHCLERDAQ
LLGSGAGHHEPYVALTQRFINEDKLNEHACEYLKTNIANSDEYTIRIFRNNAAHLSA
VRNANLYIDKLKEFKSYYEIYHFLSQENIYGKYCVDKKYVTADENGSKTISVKISKD
YCPQVYIDKSLEYFDKLNKYGTYCKDFTKALNSPFGYNLARYKNLSIEGLFDRNRPG
DKGENTFED(SEQ ID NO: 1).

### Example 3. Including similar sequence

In Example 1, the Cas 13 protein has an amino acid sequence that shares about 50% or more, at least about 51% or more, about 52% or more, about 53% or more, about 54% or more, about 55% or more, about 56% or more, about 57% or more, about 58% or more, about 59% or more, about 60% or more, about 61% or more, about 62% or more, about 63% or more, about 64% or more, about 65% or more, about 66% or more, about 67% or more, about 68% or more, about 69% or more, about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, about 99% or more, or about 100% identity, sameness, compatibility, and/or equivalence with an amino acid sequence of SEQ ID NO: 1.

### Example 4. Specification of function

In any of Example 1 to 3, the Cas 13 protein has a function of cleaving single-stranded nucleic acids.

### Example 5. Specification of PFS

In any of Example 1 to 4, the Cas 13 protein recognizes Protospacer Flanking Sequence (PFS) selected from the following sequences:
RNA sequence selected from 5'-N-3', 5'-NN-3', and 5'-NNN-3',
wherein each N is independently selected from A, U, C, or G; and
DNA sequence selected from 5'-N-3', 5'-NN-3', and 5'-NNN-3',
wherein each N is independently selected from A, T, C, or G.

### Example 6. No PFS required

In any of Example 1 to 3 to 4, when the Cas 13 protein exhibits function of cleaving single-stranded nucleic acid,
it doesn't recognize PFS, doesn't require to recognize PFS, and/or doesn't rely on PFS.

### Example 7. Including modified domain

In any of Example 1 to 6, the Cas 13 protein includes a modified protein domain.

### Example 8. Meaning of modification

In Example 7, the modified protein domain of the Cas 13 protein refers to a domain having at least one amino acid substitution, deletion, and/or insertion relative to non-modified protein domain.

### Example 9. Modified domain

In Example 7 or 8, the Cas 13 protein includes a modified HEPN domain.

### Example 10. Including additional domain

In any of Example 1 to 9, the Cas 13 protein further includes an additional protein domain.

### Example 11. Specification of nucleic acid

In any of Example 1 to 10, the single-stranded nucleic acid is single-stranded RNA, or single-stranded DNA.

### Programmable guide RNA

### Example 12. Programmable guide RNA

A programmable guide RNA comprising:
a direct repeat; and
a guide domain designed to target a predetermined target sequence,
wherein the programmable guide RNA is an RNA with the direct repeat and the guide domain linked sequentially from a 5'-end to a 3'-end direction,
the direct repeat is a region which can interact with VI-D subtype of Cas 13 protein to form a complex.

### Example 13. Description of structural formula

In Example 12, the programmable guide RNA includes the following:
5'-[direct repeat]-[guide domain]-3',
wherein the direct repeat and the guide domain are as defined in Example 12.

### Example 14. Specification of direct repeat sequence

In Example 12 or Example 13, the direct repeat has an RNA sequence of GAAACGUACUACACCCUUUUUGUAAGGGUCUGAAACC(SEQ ID NO: 2).

### Example 15. Including sequences similar to direct repeat

In Example 12, the direct repeat has an RNA sequence that shares about 50% or more, about 51% or more, about 52% or more, about 53% or more, about 54% or more, about 55% or more, about 56% or more, about 57% or more, about 58% or more, about 59% or more, about 60% or more, about 61% or more, about 62% or more, about 63% or more, about 64% or more, about 65% or more, about 66% or more, about 67% or more, about 68% or more, about 69% or more, about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, about 99% or more, or about 100% identity, sameness, compatibility, and/or equivalence with an RNA sequence of GAAACGUACUACACCCUUUUUGUAAGGGUCUGAAACC(SEQ ID NO: 2).

### Example 16. Specification of direct repeat function

In any of Example 12 to 15, the direct repeat can interact with the Cas 13 protein of any of Example 1 to 11, and for a complex.

### Example 17. Specification of length

In any of Example 12 to 16, the guide domain has a length selected from 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, and 50 nt, or it has a length within a selected range of two values from the aforementioned numerical values (e.g., 17 nt to 20 nt).

### Example 18. Meaning of target

In any one of Examples 12 to 17, the guide domain can complementarily bind to, and/or hybridize with, a nucleic acid of the predetermined target sequence.

### Example 19. Specification of percentage of complementary sequence

In any of Example 12 to 18, the guide domain has a sequence that shares about 50% or more, about 51% or more, about 52% or more, about 53% or more, about 54% or more, about 55% or more, about 56% or more, about 57% or more, about 58% or more, about 59% or more, about 60% or more, about 61% or more, about 62% or more, about 63% or more, about 64% or more, about 65% or more, about 66% or more, about 67% or more, about 68% or more, about 69% or more, about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, about 99% or more, or about 100% complementarity with the predetermined target sequence.

### Example 20. Specification of mismatch number of complementary sequence

In any one of Examples 12 to 19, the guide domain includes a complementary sequence to the predetermined target sequence, having 0, 1, 2, 3, 4, 5, 6, 7, 8, 9, 10, 11, 12, 13, 14, or 15 mismatches.

### Example 21. Specification of target RNA

In any one of Examples 12 to 20, a nucleic acid of the predetermined target sequence is RNA or DNA.

### Cas 13 complex

### Example 22. Cas 13 complex

A CRISPR/Cas 13 complex including the following:
a Cas 13 protein of any one of Examples 1 to 11; and
a programmable guide RNA of any one of Examples 12 to 21
wherein the Cas 13 protein interacts with the direct repeat of the programmable guide RNA to form a complex.

### Example 23. Recognition of PFS and target nucleic acid

In Example 22, the CRISPR/Cas 13 complex can bind to a target nucleic acid, and the target nucleic acid sequence includes the following sequence:
A predetermined target sequence that shares about 50% or more, about 51% or more, about 52% or more, about 53% or more, about 54% or more, about 55% or more, about 56% or more, about 57% or more, about 58% or more, about 59% or more, about 60% or more, about 61% or more, about 62% or more, about 63% or more, about 64% or more, about 65% or more, about 66% or more, about 67% or more, about 68% or more, about 69% or more, about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, about 99% or more, or about 100% complementary with a sequence of the guide domain of the programmable guide RNA.

### Example 24. Including PFS

In Example 23, when the Cas 13 protein of the CRISPR/Cas 13 complex recognizes the PFS, a sequence of the target nucleic acid further includes the Protospacer Flanking Sequence (PFS) recognized by the Cas 13 protein.

### Example 25. Specification of PFS

In Example 24, the PFS is selected from the following:
an RNA sequence selected from 5'-N-3', 5'-NN-3', and 5'-NNN-3'
wherein each N is independently selected from A, U, C, or G; and
a DNA sequence selected from 5'-N-3', 5'-NN-3', and 5'-NNN-3'
wherein each N is independently selected from A, T, C, or G.

### Example 26. Relative position between PFS and target sequence

In any one of Examples 23 to 25, the PFS in the target nucleic acid is adjacent to a 3'-end or a 5'-end of the target sequence.

### Example 27. Distance from PFS to target sequence

In Examples 26, the PFS is apart from the target sequence by 0 nt, 1 nt, 2 nt, or 3 nt.

### Example 28. Cas 13 complex, collateral cleavage function

In any one of Examples 22 to 27, the CRISPR/Cas 13 complex has a collateral cleavage function, and
when the CRISPR/Cas 13 complex binds to the target nucleic acid, the collateral cleavage function is activated, and cleaves any nucleic acids neighboring the CRISPR/Cas 13 complex.

### Example 29. Cas 13 complex, target nucleic acid cleavage function

In any one of Examples 22 to 28, the CRISPR/Cas 13 complex has a target specific cleavage function, and
when the CRISPR/Cas 13 complex binds to the target nucleic acid, it cleaves the target nucleic acid.

### Example 30. Specification of type of nucleic acid

In any one of Examples 28 to 29, the target nucleic acid and any nucleic acid are RNA or DNA.

### Vector being able to express each component of CRISPR/Cas 13 system

### Example 31. Expression vector

A vector capable of expressing components of CRISPR/Cas 13 system, comprising the following:
a nucleic acid encoding the Cas 13 protein of any of Example 1 to 11; and
a nucleic acid encoding the programmable guide RNA of Example 12 to 21.

### Example 32. Included in One vector

In Example 31, the nucleic acid encoding the Cas 13 protein and the nucleic acid encoding the programmable guide RNA are included in one nucleic acid molecule.

### Example 33. Included in Different vectors

In Example 31, the nucleic acid encoding the Cas 13 protein and the nucleic acid encoding the programmable guide RNA are included in separate nucleic acid molecules.

### Example 34. Type of vector

In any one of Examples 31 to 33, the vector is non-viral vector, and/or viral vector.

### Example 35. Type of non-viral vector

In Example 34, the non-viral vector is selected from plasmid, phage, naked DNA, DNA complex, mRNA, and PCR amplicon.

### Example 36. Type of viral vector

In Examples 34 and 35, the viral vector is selected from retroviral vector, lentiviral vector, adenovirus, adeno-associated virus, vacciniavirus, pox virus and Herpes Simplex Virus (HSV).

### Example 37. Specification of type of nucleic acid

In any one of Examples 31 to 36, the nucleic acid encoding the Cas 13 protein and the nucleic acid encoding the programmable guide RNA are each independently selected from DNA or RNA.

### Example 38. DNA encoding Cas 13 protein

In Example 37, the nucleic acid encoding the Cas 13 protein has DNA sequence of

GTGAGCAAGAATGATAACATCAAATCCAAAGCAAAAGCACTTGGCTTGAAGTCAACG

TTTCAGGTTGGCGACGAGGTCGTTATGACTTCTTTCGGTAAAGGTAATAAAGCCATC

GTTGAAAAGATTGTACGTGGAACCGACGTGCAGTCTGTTCCGACTGAGCCGAACTTC

AGCGCTGAAATCGAAGGTAAAAAATTTGACCTTGTCGGCAGAGCTCATATTCAGACA

AAGTCGGACAATCCTCAGTATTCAAAGAAAAGAACCGGCGACGATATGATAGGCGCA

AAGGCGGCACTTGAAAAGAGATTCTTCGGCGGTACGTTTGACGACAATATACATATT

CAGCTCGCTTACAATGTTCTTGATATTGAAAAGATTCTTTCTGTCCATATCAACAAT

ATTGTTTACACGCTTAATAACATACGAAGAAAAGACAACGCTGAAGATGACGATTTT

ATCGGCTATATGAGTACCAGAAACGACTACGACACCTTTATTGAACCGAGAAAGCAC

AACATTAGCGAAGACGCTGCTAAAAGCATTGACAAATCAAGAGCCTCGTTTGAAGAG

TATCTTCAGCCGCCGGTAAAGGATTGTCTGCATTATTTCGGAAACACCTTTTTTGCT

CCCCGTGAGATTGAAAAGACATACACGGATAATCGCGGCTTTGAAAGGAAGAAAAAA

GTTACCGTAAACTCGCTTATAGACGAGAAGGAAATATACTATATTTTTGCCCTCCTC

GGCGGACTTCGTCAATTCTGTACCCATGACAACAAAAGCGGCAGAAACTGGCTTTAT

TCTCTCGAGGAAAACGGAATAAACTCTGATGCAAAGGCGGTACTTGACAAGTACTAT

AATTCTGCAGTTGCAAGGATTGACGAAAGCTTTGTTGACAACGCATCTAAAACTAAT

TTCAAGCTGATTTTTAATGCAATGGATGTCAGCGACGAAGCGCTTCAGGACAATATA

GCAAAGGCGTTTTACAAGTTTACGGTTTGTAAGAGCTTTAAAAACATGGGCTTTTCA

ATTAAAAAGCTCCGTGAACAGCTGCTCGAACTTCCTGAATATGAAGGGCTTAAAGAT

AAGCATTACGATTCCGTGCGCTCAAAGCTTTATCAGATTATGGACTTCGTTATTTAT

CTGAGTTTCAAAGATGAAAAGTTTAAGAAGGATAATGAAGAAAAGATTAATAATATC

GTTAACGAACTCCGTGCGACCTTGAACGAAGAGGATAAAGGCAGAGTATATGCCTCT

TATGCCGAAAGGATGAAATCTGAGCTTAAACCGGCAATTGCACGCCTTAAGTCTGAT
ATTGACAAAATTAAGGACAGCAGGGTGAAAGAGTTTGAACTTGATGCGTCCGTAAAA
TATAGACTCTCAAAGGTCGTAGAAAGTGTGCGCCTTAAGGATAGGGCAACATATTTT
ACAAAGCTCATATATCTGACTACTCTGTTCCTTGACGGCAAGGAGATTAACGACCTC
CTGACTACTCTTATTCATCAGTTTGAGAATATCGCAAGTTTTATTGATGTTATGAAC
GACAGAGGTATTGATTGCCGTTTTTCGGATGGATATAAGCTTTTTGAATCCTCAAAG
CAAATTGCGTTTGAGCTTCGCAATGTCAATAGCTTTGCTCGTATGACAAGGACCTCA
AAGGATGACGAAAACGCAACGCATATGATGTATATAGATGCTGCAGAGATTCTCGGC
ACCGATTATACCGAAGAGCAGATTGAGGAGCATCTTAACCTGGAAAAGAAAAGAATG
ATACCAGGCACGAAAAAAGCGGATATGAACTTCCGTAACTTTATAATCAATAACGTT
ATAAAGTCATCACGCTTCAATTATCTTGTCAGATATTCCAATCCTAAAAAAATCAGA
GCGCTCGCCGATAACGAGGGTGTAATCCGTTTTGTCCTGGGCGAGTTGCCGGATGCG
CAGATTGACCGTTATACTTTACTTTGCGGCTTCAATCCCGATGCCGACCGGCAGGAA
AAAACGGACAAACTTGCAAAGGCAATTACTGGTTTGAGGTTTAACGATTTTGAAAAT
GTTAAACAGGGCGCAAATACTGAGGGCGAATCTCAGGAGTCAATTGACAAGGCACAG
AAGCAGGGACTGATTTCTTTGTACTTAACCGTTCTATATCTTTTGACAAAGAATCTT
GTTTATGTAAACTCACGCTATTTTCTTGCATTCCATTGTCTTGAGCGTGATGCGCAG
CTGCTTGGAAGCGGTGCTGGGCATCATGAGCCTTATGTTGCTCTTACTCAGCGCTTT
ATTAATGAAGATAAGCTCAATGAGCATGCCTGCGAATATCTTAAGACCAATATTGCA
AATTCGGATGAATATACAATTAGAATATTCCGCAATAATGCTGCTCACTTGAGTGCT
GTTAGAAATGCAAATCTGTATATTGACAAGTTGAAGGAATTTAAATCGTACTATGAG
ATTTATCATTTCCTTTCTCAAGAGAATATTTACGGTAAATATTGCGTTGATAAGAAA
TATGTTACAGCCGATGAAAATGGAAGTAAAACAATAAGTGTTAAAATTAGCAAGGAT
TATTGTCCTCAGGTATATATCGACAAGTCGCTTGAATACTTTGACAAGCTCAATAAA
TATGGTACGTATTGCAAGGACTTCACGAAGGCACTCAATTCGCCATTCGGCTACAAC

CTCGCAAGATATAAGAATCTGTCTATCGAGGGCTTGTTTGACCGAAACCGTCCCGGA

GACAAAGGTGAGAACACGTTTGAGGACTAA(SEQ ID NO: 4).

### CRISPR/Cas 13 composition

### Example 39. CRISPR/Cas 13 composition

A CRISPR/Cas 13 composition comprising the following:
the Cas 13 protein of any one of Example 1 to 11, or a nucleic acid encoding the Cas 13 protein; and
the programmable guide RNA of any one of Example 12 to 21, or a nucleic acid encoding the programmable guide RNA.

### Example 40. CRISPR/Cas 13 complex

In Example 39, the CRISPR/Cas 13 composition includes the Cas 13 protein and the programmable guide RNA, where the Cas 13 protein binds to the programmable guide RNA to form a ribonucleoprotein.

### Example 41. Specification of complex

In Example 40, the CRISPR/Cas 13 composition includes the CRISPR/Cas 13 complex of any one of Example 22 to 30.

### Example 42. Expression vector for component of CRISPR/Cas 13 system

In Example 39, the CRISPR/Cas 13 composition includes the nucleic acid encoding the Cas 13 protein and the nucleic acid encoding the programmable guide RNA.

### Example 43. Specification of expression vector

In Example 42, the CRISPR/Cas 13 composition includes expression vectors for each component of the CRISPR/Cas 13 system of any one of Examples 31 to 38

### Example 44. Nucleic acid encoding protein and guide RNA

In Example 39, the CRISPR/Cas 13 composition includes the nucleic acid encoding the Cas 13 protein and the programmable guide RNA

### Example 45. Specification of mRNA

In Example 44, the nucleic acid encoding the Cas 13 protein is mRNA.

### Composition for detecting target nucleic acid

### Example 46. Composition for detecting target nucleic acid

A detecting agent comprising the following:
a reporter including a probe nucleic acid,
wherein when the probe nucleic acid is cleaved, the reporter generates a detectable signal.

### Example 47. Specification of detectable signal

In Example 46, the detectable signal is a fluorescence signal.

### Method of cleaving non-target nucleic acids/Use

### Example 48. Method of cleaving non-target nucleic acids

A method of cleaving non-target nucleic acids using a CRISPR/Cas 13 system, comprising:
a step of contacting the CRISPR/Cas 13 complex in any one of Examples 22 to 30, with a target nucleic acid and non-target nucleic acids,
wherein the target nucleic acid has 1) a predetermined target sequence, and 2) a PFS recognized by the Cas 13 protein of the CRISPR/Cas 13 complex,
the guide domain of the programmable guide RNA of the CRISPR/Cas 13 complex is designed to target the predetermined target sequence,
the non-target nucleic acids have a sequence different from that of the target nucleic acid, and
wherein, as a result of the CRISPR/Cas 13 complex contacting the target nucleic acid, a collateral cleavage activity is activated, and the non-target nucleic acid is cleaved.

### Example 49. Order of contacting

In Example 48, the step of contacting the CRISPR/Cas 13 complex with the target nucleic acid and the non-target nucleic acids is performed in an order selected from the following:
(a) contacting the CRISPR/Cas 13 complex with the target nucleic acid, followed by contacting it with the non-target nucleic acids;
(b) contacting the CRISPR/Cas 13 complex with the non-target nucleic acids, followed by contacting it with the target nucleic acid; and
(c) contacting the CRISPR/Cas 13 complex with the target nucleic acid and the non-target nucleic acids simultaneously.

### Example 50. Use of non-target nucleic acids cleavage

Use of the CRISPR/Cas 13 complex of any one of Examples 22 to 30, the expression vector for components of the CRISPR/Cas 13 system of any one of Examples 31 to 38, and/or the CRISPR/Cas 13 composition of any one of Examples 39 to 45 in the method of cleaving non-target nucleic acids of any one of Examples 48 to 49.

### Method of detecting target nucleic acid/Use

### Example 51. Method of detecting target nucleic acid

A method of detecting a target nucleic acid using a CRISPR/Cas 13 system comprising the following:
(a) a step of blending, mixing, reacting, and/or contacting a sample, the CRISPR/Cas 13 complex of any one of Examples 22 to 30, and the detecting agent of any one of Examples 46 to 47,
   wherein the target nucleic acid includes 1) a predetermined target sequence, and 2) a PFS recognized by the Cas 13 protein of the CRISPR/Cas 13 complex,
   the guide domain of the programmable guide RNA of the CRISPR/Cas 13 complex is designed to target the predetermined target sequence,
   when the target nucleic acid is present in the sample, the target nucleic acid binds to the CRISPR/Cas 13 complex, and leading to activating a collateral cleavage activity, thereby enabling the probe nucleic acid included in the reporter to be cleaved by the Cas 13 complex, and
   when the probe nucleic acid included in the reporter of the detecting agent is cleaved, the reporter generates a detectable signal; and
(b) a step of observing, measuring, and/or detecting the detectable signal generated from the mixture,
wherein the presence or absence and/or the amount of the target nucleic acid present in a sample can be derived, found out, measured, determined, detected, and/or analyzed from the detectable signal.

### Example 52. Order of contacting sample, complex and detecting agent

In Example 51, the step of blending, mixing, reacting, and/or contacting a sample, the CRISPR/Cas 13 complex of any one of Examples 22 to 30, and the detecting agent of any one of Examples 46 to 47 is performed in an order selected from the following:
(1) blending, mixing, reacting, and/or contacting the sample and the CRISPR/Cas 13 complex, followed by blending, mixing, reacting, and/or contacting the detecting agent;
(2) blending, mixing, reacting, and/or contacting the sample, and the detecting agent, followed by blending, mixing, reacting, and/or contacting the CRISPR/Cas 13 complex;
(3) blending, mixing, reacting, and/or contacting the CRISPR/Cas 13 complex, and the detecting agent, followed by blending, mixing, reacting, and/or contacting the sample; and
(4) simultaneously blending, mixing, reacting, and/or contacting the sample, the CRISPR/Cas 13 complex, and the detecting agent.

### Example 53. Method of detecting COVID-19

In any one of Examples 51 to 52, the method of detecting the target nucleic acid using the CRISPR/Cas 13 system is a method of detecting whether the nucleic acid of the COVID-19 virus is present in the sample.

### Example 54. Method of detecting COVID-19 N-gene, E-gene

In Example 53, the target nucleic acid is

ucugauaauggaccccaaaaucagcgaaaugcaccccgcauuacguuugguggaccc
ucagauucaacuggcaguaaccagaauggagaacgcaguggggcgcgaucaaaacaa
cgucggccccaagguuuacccaauaauacugcgucuugguucaccgcucucacucaa
cauggcaaggaagaccuuaaauucccucgaggacaaggcguuccaauuaacaccaau
agcaguccagaugaccaaauuggcuacuaccgaagagcuaccagacgaauucguggu
ggugacgguaaaaugaaagaucucaguccaagaugguauuucuacuaccuaggaacu
gggccagaagcuggacucccuauggugcuaacaaagacggcaucauaggguugcaac
ugagggagccuugaauacaccaaaagaucacauuggcacccgcaauccugcuaacaa
ugcugcaaucgugcuacaacuuccucaaggaacaacauugccaaaaggcuucuacgc
agaagggagcagaggcggcagucaagccucuucucguuccucaucacguagucgcaa
caguucaagaaauucaacuccaggcagcaguaggggaaccuccugcuagaauggcug
gcaauggcggugaugcugcucuugcuuugcugcugcuugacagauugaaccagcuug
agagcaaaaugucugguaaaggccaacaacaacaaggccaaacugucacuaagaaau
cugcugcugaggcuucuaagaagccucggcaaaaacguacugccacuaaagcauaca
auguaacacaagcuuucggcagacgugguccagaacaaacccaaggaaauuuugggg
accaggaacuaaucagacaaggaacugauuacaaacauuggccgcaaauugcacaau
uugcccccagcgcuucagcguucuucggaaugucgcgcauuggcauggaagaaaaa
(SEQ ID NO: 5) and/or

uacucauucguuucggaagagacagguacguuaauaguuaauagcguacuucuuuuu
cuugcuuucgugguauucuugcuaguuacacuagccauccuuacugcgcuucgauug
ugugcguacugcugcaauauuguuaacgugagucuuguaaaaccuucuuuuuacguu
uacucucguguuaaaaaucugaauucuucuagaguuccugaucuucuggucuaaaaa
a(SEQ ID NO: 6).

### Example 55. Specification of target sequence

In Example 54, a target sequence of the target nucleic acid is a nucleic acid selected from CGCAUUACGUUUGGUGGACC(SEQ ID NO: 57), UUCAACUGGCAGUAACCAGA(SEQ ID NO: 58), UUACAAACAUUGGCCGCAAA(SEQ ID NO: 59), GGGAGCCUUGAAUACACCAA(SEQ ID NO: 60), CACAUUGGCACCCGCAAUCC(SEQ ID NO: 61), AAUGCUGCAAUCGUGCUACA(SEQ ID NO: 62), GGGGAACUUCUCCUGCUAGA(SEQ ID NO: 63), CUUUGCUGCUGCUUGACAGA(SEQ ID NO: 64), CAGCUUGAGAGCAAAAUGUC(SEQ ID NO: 65), GAUUCAACUGGCAGUAACCA(SEQ ID NO: 66), CACAUUGGCACCCGCAAUCCU(SEQ ID NO: 67), AAUGCUGCAAUCGUGCUACAA(SEQ ID NO: 68), UUGGCCGCAAAUUGCACAAUUUG(SEQ ID NO: 69), AGGAACUGAUUACAAACAUUGGC(SEQ ID NO: 70), GGGACCAGGAACUAAUCAGACAA(SEQ ID NO: 71), CUAGCCAUCCUUACUGCG(SEQ ID NO: 72), CGGAAGAGACAGGUACGUUA(SEQ ID NO: 73), GUUACACUAGCCAUCCUUAC(SEQ ID NO: 74), UGUGCGUACUGCUGCAAUAUU(SEQ ID NO: 75), and CAAUAUUGUUAACGUGAGUCUU(SEQ ID NO: 76).

### Example 56. Specification of guide domain

In Example 54, a guide domain of the programmable guide RNA of the CRISPR/Cas 13 complex has a nucleic acid sequence selected from ggUccaccaaacgUaaUgcg(SEQ ID NO: 37), UCUGGUUACUGCCAGUUGAA(SEQ ID NO: 38), UUUgcggccaaUgUUUgUaa(SEQ ID NO: 39), UUGGUGUAUUCAAGGCUCCC(SEQ ID NO: 40), GGAUUGCGGGUGCCAAUGUG(SEQ ID NO: 41), UGUAGCACGAUUGCAGCAUU(SEQ ID NO: 42), UCUAGCAGGAGAAGUUCCCC(SEQ ID NO: 43), UCUGUCAAGCAGCAGCAAAG(SEQ ID NO: 44), GACAUUUUGCUCUCAAGCUG(SEQ ID NO: 45), UggUUacUgccagUUgaaUc(SEQ ID NO: 46), aggaUUgcgggUgccaaUgUg(SEQ ID NO: 47), UUgUagcacgaUUgcagcaUU(SEQ ID NO: 48), CAAAUUGUGCAAUUUGCGGCCAA(SEQ ID NO: 49), GCCAAUGUUUGUAAUCAGUUCCU(SEQ ID NO: 50), UUGUCUGAUUAGUUCCUGGUCCC(SEQ ID NO: 51), cgcagUaaggaUggcUag(SEQ ID NO: 52), UaacgUaccUgUcUcUUccg(SEQ ID NO: 53), gUaaggaUggcUagUgUaac(SEQ ID NO: 54), aaUaUUgcagcagUacgcaca(SEQ ID NO: 55), and aagacUcacgUUaacaaUaUUg(SEQ ID NO: 56).

### Example 57. Specification of programmable guide RNA

In Example 54, the programmable guide RNA in the CRISPR/Cas 13 complex has a nucleic acid sequence selected from
GAAACGUACUACACCCUUUUUGUAAGGGUCUGAAACCUggUUacUgccagUUgaaUc (SEQ ID NO: 16), 
GAAACGUACUACACCCUUUUGUAAGGUCUGAAACCaggaUUgcgggUgccaaUgU
g(SEQ ID NO: 17),
 
GAAACGUACUACACCCUUUUGUAAGGUCUGAAACCUUgUagcacgaUUgcagcaU
U(SEQ ID NO: 18),
 
GAAACGUACUACACCCUUUUGUAAGGGUCUGAAACCCAAUUGUGCAAUUUGCGGC
CAA(SEQ ID NO: 19),
 
GAAACGUACUACACCCUUUUGUAAGGUCUGAAACCGCCAAUGUUUGUAAUCAGUU
CCU(SEQ ID NO: 20),
 
GAAACGUACUACACCCUUUUGUAAGGUCUGAAACCUUGUCUGAUUAGUUCCUGGU
CCC(SEQ ID NO: 21),
GAAACGUACUACACCCUUUUUGUAAGGGUCUGAAACCggUccaccaaacgUaaUgcg (SEQ ID NO: 22),
GAAACGUACUACACCCUUUUUGUAAGGGUCUGAAACCUCUGGUUACUGCCAGUUGAA (SEQ ID NO: 23),
GAAACGUACUACACCCUUUUUGUAAGGGUCUGAAACCUUUgcggccaaUgUUUgUaa (SEQ ID NO: 24),
GAAACGUACUACACCCUUUUUGUAAGGGUCUGAAACCUUGGUGUAUUCAAGGCUCCC (SEQ ID NO: 25),
GAAACGUACUACACCCUUUUUGUAAGGGUCUGAAACCGGAUUGCGGGUGCCAAUGUG (SEQ ID NO: 26),
GAAACGUACUACACCCUUUUUGUAAGGGUCUGAAACCUGUAGCACGAUUGCAGCAUU (SEQ ID NO: 27),
GAAACGUACUACACCCUUUUUGUAAGGGUCUGAAACCUCUAGCAGGAGAAGUUCCCC (SEQ ID NO: 28),
GAAACGUACUACACCCUUUUUGUAAGGGUCUGAAACCUCUGUCAAGCAGCAGCAAAG (SEQ ID NO: 29),
GAAACGUACUACACCCUUUUUGUAAGGGUCUGAAACCGACAUUUUGCUCUCAAGCUG (SEQ ID NO: 30),
GAAACGUACUACACCCUUUUUGUAAGGGUCUGAAACCUaacgUaccUgUcUcUUccg (SEQ ID NO: 32),
GAAACGUACUACACCCUUUUUGUAAGGGUCUGAAACCgUaaggaUggcUagUgUaac (SEQ ID NO: 33),

GAAACGUACUACACCCUUUUUGUAAGGGUCUGAAACCaaUaUUgcagcagUacgcac
a(SEQ ID NO: 34),

GAAACGUACUACACCCUUUUUGUAAGGGUCUGAAACCaagacUcacgUUaacaaUaU
Ug(SEQ ID NO: 35), and

GAAACGUACUACACCCUUUUUGUAAGGGUCUGAAACCcgcagUaaggaUggcUag(S

EQ ID NO: 36)

### Example 58. Use of detecting target nucleic acid

Use of the CRISPR/Cas 13 complex of any one of Examples 22 to 30, the expression vector for a CRISPR/Cas 13 system component of any one of Examples 31 to 38, and/or the CRISPR/Cas 13 composition of any one of Examples 39 to 45, in the method for detecting a target nucleic acid of any one of Examples 51 to 57.

### Experimental Example

Below, the present invention provided by this specification will be described in more detail through Experimental Examples and embodiments. These Examples are only intended to illustrate the content disclosed in this specification, and it will be apparent to those skill in the art that the scope of the content disclosed herein is not to be construed as limited by these examples.

### Experimental Example 1. Experimental process and material

### Experimental Example 1.1. Identification of novel CRISPR/Cas 13 system sequence from public database

Metagenome data of bovine stomach, published in the National Center for Biotechnology Information (NCBI) database, was utilized. Using the NGS data, a CRISPR/Cas 13 system was identified through the following methods:
(1) Sequence data were assembled to deduce genomic information of microorganisms by MEGAHIT v.1.2.9 (NGS assembly software). See Li D, Liu CM, Luo R, Sadakane K, Lam TW. MEGAHIT: an ultra-fast single-node solution for large and complex metagenomics assembly via succinct de Bruijn graph. Bioinformatics. 2015 May 15;31(10):1674-6. doi: 10.1093/bioinformatics/btv033. Epub 2015 Jan 20. PMID: 25609793.
(2) Referring to published information of Cas 13 sequence e.g., Cas 13 derived from Lachnospiraceae bacterium, Cas 13 protein candidates included in genomic information of the microorganisms were identified by using BLAST tool based on results of homology analysis.
(3) Using the same method as described in (2), guide RNA candidates for each Cas 13 protein candidate were identified.

Below, experiments were conducted using sequence information of the CRISPR/Cas 13 system identified through these methods.

Information about each component of the CRISPR/Cas 13 system identified using these methods is as follows:

**[Table 1]**

| Subject | Sequence | SEQ ID NO |
|---|---|---|
| Cas13 protein | VSKNDNIKSKAKALGLKSTFQVGDEVVMTSFGKGNKAIVEKI VRGTDVQSVPTEPNFSAEIEGKKFDLVGRAHIQTKSDNPQYS KKRTGDDMIGAKAALEKRFFGGTFDDNIHIQLAYNVLDIEKIL SVHINNIVYTLNNIRRKDNAEDDDFIGYMSTRNDYDTFIEPR KHNISEDAAKSIDKSRASFEEYLQPPVKDCLHYFGNTFFAPRE IEKTYTDNRGFERKKKVTVNSLIDEKEIYYIFALLGGLRQFCT HDNKSGRNWLYSLEENGINSDAKAVLDKYYNSAVARIDESFV DNASKTNFKLIFNAMDVSDEALQDNIAKAFYKFTVCKSFKN MGFSIKKLREQLLELPEYEGLKDKHYDSVRSKLYQIMDFVIYL SFKDEKFKKDNEEKINNIVNELRATLNEEDKGRVYASYAERM | 1 |
| | KSELKPAIARLKSDIDKIKDSRVKEFELDASVKYRLSKVVESVR LKDRATYFTKLIYLTTLFLDGKEINDLLTTLIHQFENIASFIDV MNDRGIDCRFSDGYKLFESSKQIAFELRNVNSFARMTRTSKD DENATHMMYIDAAEILGTDYTEEQIEEHLNLEKKRMIPGTKK ADMNFRNFIINNVIKSSRFNYLVRYSNPKKIRALADNEGVIRF VLGELPDAQIDRYTLLCGFNPDADRQEKTDKLAKAITGLRFN DFENVKQGANTEGESQESIDKAQKQGLISLYLTVLYLLTKNLV YVNSRYFLAFHCLERDAQLLGSGAGHHEPYVALTQRFINEDK LNEHACEYLKTNIANSDEYTIRIFRNNAAHLSAVRNANLYIDK LKEFKSYYEIYHFLSQENIYGKYCVDKKYVTADENGSKTISVKI SKDYCPQVYIDKSLEYFDKLNKYGTYCKDFTKALNSPFGYNL ARYKNLSIEGLFDRNRPGDKGENTFED | |
| Direct repeat | GAAACGUACUACACCCUUUUUGUAAGGGUCUGAAACC | 2 |

### Experimental Example 1.2. Production of expression vector for novel Cas 13 protein

For an amino acid sequence of the novel Cas 13 protein discovered in Experimental Example 1.1, a nucleic acid sequence optimized for Escherichia coli (E. Coli) codons was identified, and this nucleic acid sequence was cloned into the pET28 vector to construct expression vector for the novel Cas 13 protein.

### Experimental Example 1.3. Production and purification of novel Cas 13 protein

The expression vector for a novel Cas 13 protein produced in Experiment Example 1.2 was induced in vivo, and the novel Cas 13 protein was produced in various E. coli strains (NiCo21(DE3), T7 Express lysY/Iq, NEB^{®} Express Iq) under different growth conditions (media, temperature, induction conditions, etc.). Then, an amount of the Cas 13 protein produced was assayed through SDS-PAGE analysis.

Based on results of the assay, optimized growth conditions and strains for flask-scale production were selected.

Specifically, E. coli cells were transformed with the plasmid vectors produced in Experiment Example 1.2 according to the manufacturer's instruction. Single colonies were grown overnight at 37°C in Luria-Bertani liquid medium containing 100 µg/ml ampicillin. The cells were diluted 1:200 in 250 ml of the same medium and grown at 37°C until an OD600 reached 0.70 to 0.75. A culture was incubated at room temperature for 1 hour, and then induced to express proteins with 0.5 mM isopropyl-b-D-thiogalactopyranoside during 16 - 18 hours of incubation at 23°C with shaking at 220 rpm.

After inducing E. coli to produce the novel Cas 13 protein under the optimized growth conditions, cells were pelleted by centrifugation at ~4000g for 20 minutes, and stored at -20°C in a refrigerator.

Before purifying the novel Cas 13 protein, frozen cells were resuspended in a sonication buffer (20 mM Tris-HCl, pH 7.5, 0.5 M NaCl, 5% glycerol, 0.2% Tween20, 1 mM DTT, 1 mM EDTA, 1x Halt Protease Inhibitor Cocktail (Fisher Scientific), 40 mM imidazole) at 25°C, using 5 ml of the buffer per gram of pellet.

Pellets suspended in the sonication buffer were disrupted using a Qsonica Microtip Probe 420-A, with conditions set to 30% amplitude, 5 seconds ON/15 seconds OFF per 30 ml of a sample, on ice, for 2 minutes x 4 (performed with intervals to prevent overheating).

After the sonication, samples were centrifuged at 40,000g for 1 hour to remove cell debris.

Supernatant obtained from the above centrifugation, was filtered using a 0.2 µm PES syringe filter (Fisher Scientific) and loaded onto a Ni2+-charged HiTrap Chelating HP column (Cytiva) equilibrated with 20 mM Tris-HCl, pH 7.5 in 0.5 M NaCl, and 40 mM imidazole buffer at 25°C.

The column was eluted with a linear gradient of imidazole from 40 mM to 700 mM in 20 mM Tris-HCl, pH 7.5, and 0.5 M NaCl at 25°C, yielding the novel Cas 13 protein.

### Experimental Example 1.4. Production of programmable guide RNA

The programmable guide RNA of the novel CRISPR/Cas 13 system identified in Experimental Example 1.1 (also referred to as crRNA) was synthesized through in vitro transcription using a TranscriptAid T7 High Yield Transcription Kit (Thermo Fisher) or a HiScribe T7 Quick High Yield RNA Synthesis Kits (New England Biolabs).

For the in vitro transcription, a fragment containing T7 promoter at the proximal end of the crRNA encoding sequence was produced by PCR using primers as a template, or the T7 primer oligonucleotides were annealed to single-stranded template oligonucleotides.

The produced crRNA was purified using a RNeasy MiniElute Cleanup Kit (Qiagen) or a Monarch RNA Cleanup Kit (50 µg) (New England Biolabs).

The primers used in the in vitro transcription are as shown in the table below:

**[Table 2]**

| Primers used for synthesizing a programmable guide RNA of a novel CRISPR/Cas 13 system | | |
|---|---|---|
| Label | Sequence | SEQ ID NO |
| crRNA Forward primer | GAAATTAATACGACTCACTATAGGAAACGTACTACACCCTT TTTGTAAGGGTCTGAAACC | 77 |
| crRNA Example 2.1 Reverse primer | GATTCAACTGGCAGTAACCAGGTTTCAGACCCTTACAAAAA GGG | 78 |
| crRNA Example 2.2 Reverse primer | CACATTGGCACCCGCAATCCTGGTTTCAGACCCTTACAAAA AGGG | 79 |
| crRNA Example 2.3 Reverse primer | AATGCTGCAATCGTGCTACAAGGTTTCAGACCCTTACAAAA AGGG | 80 |
| crRNA Example 2.4 Reverse primer | TTGGCCGCAAATTGCACAATTTGGGTTTCAGACCCTTACAA AAAGGG | 81 |
| crRNA Example 2.5 Reverse primer | AGGAACTGATTACAAACATTGGCGGTTTCAGACCCTTACAA AAAGGG | 82 |
| crRNA Example 2.6 Reverse primer | GGGACCAGGAACTAATCAGACAAGGTTTCAGACCCTTACAA AAAGGG | 83 |
| crRNA Example 3.1 Reverse primer | CGGAAGAGACAGGTACGTTAGGTTTCAGACCCTTACAAAAA GGG | 84 |
| crRNA Example 3.2 Reverse primer | GTTACACTAGCCATCCTTACGGTTTCAGACCCTTACAAAAA GGG | 85 |
| crRNA Example 3.3 Reverse primer | TGTGCGTACTGCTGCAATATTGGTTTCAGACCCTTACAAAA AGGG | 86 |
| crRNA Example 3.4 Reverse primer | CAATATTGTTAACGTGAGTCTTGGTTTCAGACCCTTACAAA AAGGG | 87 |

### Experimental Example 1.5. Production of novel CRISPR/Cas 13 complex

The new CRISPR/Cas 13 complex was produced by incubating 40 nM of the Cas 13 protein produced in Experiment Example 1.3 and 40 nM of the crRNA produced in Experiment Example 1.4 in NEB r2.1 buffer containing 50 mM NaCl, 10 mM Tris-HCl, 10 mM MgCl2, 100 µg/mL BSA (pH = 7.9) at room temperature for 15 minutes.

### Experimental Example 1.6. Method of detecting target nucleic acid using a CRISPR/Cas 13 complex

A reaction solution was produced by mixing 1x NEB 2.0 buffer, 0.6 U/µL of RNase inhibitor, 12.5 nM of RNase alert (IDT reporter), target RNA at various concentrations (0.1 - 100 ng/µL) according to Experimental Examples, and 100 µL of the nuclease-free purified water.

The novel CRISPR/Cas 13 complex produced in Experiment Example 1.5 was added to the reaction solution.

The mixture was loaded into a 384-well plate (SPL) and incubated at 37°C for up to 1 hour in fluorescence plate reader (TECAN, Infinite 200 Pro M Plex). Then fluorescence detections were performed every 3 minutes under the following conditions: λex = 535 nm; λem = 485 nm, gain = -135.

### Experimental Example 2. Detection experiment 1 of target nucleic acid using novel CRISPR/Cas 13 complex

### Experimental Example 2.1. Detection experiment of target nucleic acid according to guide domain sequence

To confirm whether the novel CRISPR/Cas 13 complex produced in Experiment Example 1.5 has a collateral cleavage activity, and can be utilized for detecting a target nucleic acid, a target nucleic acid detection experiment was conducted according to Experiment Example 1.6.

The target nucleic acid corresponds to a N-gene of COVID-19, and its sequence is as follows:

ucugauaauggaccccaaaaucagcgaaaugcaccccgcauuacguuugguggaccc

ucagauucaacuggcaguaaccagaauggagaacgcaguggggcgcgaucaaaacaa
cgucggccccaagguuuacccaauaauacugcgucuugguucaccgcucucacucaa
cauggcaaggaagaccuuaaauucccucgaggacaaggcguuccaauuaacaccaau
agcaguccagaugaccaaauuggcuacuaccgaagagcuaccagacgaauucguggu
ggugacgguaaaaugaaagaucucaguccaagaugguauuucuacuaccuaggaacu
gggccagaagcuggacucccuauggugcuaacaaagacggcaucauaggguugcaac
ugagggagccuugaauacaccaaaagaucacauuggcacccgcaauccugcuaacaa
ugcugcaaucgugcuacaacuuccucaaggaacaacauugccaaaaggcuucuacgc
agaagggagcagaggcggcagucaagccucuucucguuccucaucacguagucgcaa
caguucaagaaauucaacuccaggcagcaguaggggaaccuccugcuagaauggcug
gcaauggcggugaugcugcucuugcuuugcugcugcuugacagauugaaccagcuug
agagcaaaaugucugguaaaggccaacaacaacaaggccaaacugucacuaagaaau
cugcugcugaggcuucuaagaagccucggcaaaaacguacugccacuaaagcauaca
auguaacacaagcuuucggcagacgugguccagaacaaacccaaggaaauuuugggg
accaggaacuaaucagacaaggaacugauuacaaacauuggccgcaaauugcacaau
uugcccccagcgcuucagcguucuucggaaugucgcgcauuggcauggaagaaaaaa
(SEQ ID NO: 5).

Each component of the novel CRISPR/Cas 13 complex used in Experiment Example 2.1 is set forth in the following table.

As a negative control for each experiment, only the Cas 13 protein was added without the programmable guide RNA.

**[Table 3]**

| Each component of novel CRISPR/Cas 13 complex used in Experimental Example 2 | | | | |
|---|---|---|---|---|
| Label | Cas13 protein (SEQ ID NO) | Direct Repeat (SEQ ID NO) | Guide domain | PFS |
| Example 2.1 | 1 | 2 | TGGTTACTGCCAGTTGAATC | G |
| Example 2.2 | | | AGGATTGCGGGTGCCAATGTG | G |
| Example 2.3 | | | TTGTAGCACGATTGCAGCATT | C |
| Example 2.4 | | | CAAATTGTGCAATTTGCGGCCAA | C |
| Example 2.5 | | | GCCAATGTTTGTAATCAGTTCCT | C |
| Example 2.6 | | | TTGTCTGATTAGTTCCTGGTCCC | G |

The above experimental results are shown in Figures 1 to 6:
The results demonstrate that the novel CRISPR/Cas 13 complex (1) activates collateral cleavage function when guide domains of various sequences bind to a target nucleic acid, and (2) exhibits sufficient collateral cleavage activity to detect the target nucleic acid.

### Experimental Example 2.2. Detection experiment of target nucleic acid at various concentrations

To confirm whether the novel CRISPR/Cas 13 complex produced in Experiment Example 1.5 has a collateral cleavage activity and can be utilized for detecting a target nucleic acid, detection experiment of the target nucleic acid was conducted according to Experiment Example 1.6 using the aforementioned COVID-19 N-gene as the target nucleic acid.

Each component of the novel CRISPR/Cas 13 complex used in Experiment Example 2 is set forth in the following table.

**[Table 4]**

| Each component of novel CRISPR/Cas 13 complex used in Experimental Example 2.2, and concentration | | | | | |
|---|---|---|---|---|---|
| Label | Cas13 protein (SEQ ID NO) | Direct Repeat (SEQ ID NO) | Guide domain | PFS | Concentration (ng/uL) |
| Example 2.2.1 | 1 | 2 | AGGATTGCGGGTGCCAATGTG | G | 0.05 |
| Example 2.2.2 | | | | | 0.1 |
| Example 2.2.3 | | | | | 0.25 |
| Example 2.2.4 | | | | | 0.5 |
| Example 2.2.5 | | | | | 1 |
| Example 2.5.1 | | | GCCAATGTTTGTAATCAGTTCCT | C | 0.05 |
| Example 2.5.2 | | | | | 0.1 |
| Example 2.5.3 | | | | | 0.25 |
| Example 2.5.4 | | | | | 0.5 |
| Example 2.5.5 | | | | | 1 |

As a negative control for each experiment, the Cas 13 protein was added only without the programmable guide RNA

The experimental results are shown in Figures 7 and 8:
The results demonstrate that the novel CRISPR/Cas 13 complex (1) binds to a target nucleic acid event at low concentration and activates a collateral cleavage function, and (2) exhibits a sufficient collateral cleavage activity to detect the target nucleic acid.

### Experimental Example 3. Detection experiment 2 of target nucleic acid using novel CRISP/Cas 13 complex

### Experimental Example 3.1. Detection experiment of target nucleic acid according to guide domain sequence

To confirm whether the novel CRISPR/Cas 13 complex produced according to Experiment Example 1.5 has a collateral cleavage activity and can be utilized for detecting the target nucleic acid, a target nucleic acid detection experiment was conducted according to Experiment Example 1.6.

Each component of the novel CRISPR/Cas 13 complex used in Experiment Example 3.1 is set forth in the following table.

The target nucleic acid corresponds to E-gene of COVID-19, and its sequence is as follows:

uacucauucguuucggaagagacagguacguuaauaguuaauagcguacuucuuuuu

cuugcuuucgugguauucuugcuaguuacacuagccauccuuacugcgcuucgauug

ugugcguacugcugcaauauuguuaacgugagucuuguaaaaccuucuuuuuacguu

uacucucguguuaaaaaucugaauucuucuagaguuccugaucuucuggucuaaaaa

a(SEQ ID NO: 6)

**[Table 5]**

| Each component of novel CRISPR/Cas 13 complex used in Experimental Example 3 | | | | |
|---|---|---|---|---|
| Label | Cas13 protein (SEQ ID NO) | Direct Repeat (SEQ ID NO) | Guide domain | PFS |
| Example 3.1 | 1 | 2 | TAACGTACCTGTCTCTTCCG | A |
| Example 3.2 | | | GTAAGGATGGCTAGTGTAAC | T |
| Example 3.3 | | | AATATTGCAGCAGTACGCACA | G |
| Example 3.4 | | | AAGACTCACGTTAACAATATTG | C |

The above experimental results are shown in Figures 9 to 11, and
in the case of Example 3.4, the fluorescence signal intensity exceeded the detection range immediately after the reaction was completed, and a graph was not illustrated separately.

The results demonstrate that the novel CRISPR/Cas 13 complex (1) activates the collateral cleavage function by binding all guide domains of various sequence to the target nucleic acid, (2) exhibits sufficient collateral cleavage activity to use in detecting the target nucleic acid, and (3) functions only through a presence of a complementary sequence to the guide domain, without reliance on a Protospacer Flanking Sequence.

### Experimental Example 3.2. Detection experiment of target nucleic acid at various concentrations

To confirm whether the novel CRISPR/Cas 13 complex produced according to Experiment Example 1.5 has a collateral cleavage activity and can be utilized for detecting the target nucleic acid, the target nucleic acid detection experiment was conducted using E-gene of COVID-19 as the target nucleic acid according to Experiment Example 1.6.

Each component of the novel CRISPR/Cas 13 complex used in Experiment Example 3.2 is set forth in the following table:

**[Table 6]**

| Each component of novel CRISPR/Cas 13 complex used in Experimental Example 2.2 and concentration | | | | | |
|---|---|---|---|---|---|
| Label | Cas13 protein (SEQ ID NO) | Direct Repeat (SEQ ID NO) | Guide domain | PFS | Concentration (ng/uL) |
| Example 3.3.1 | 1 | 2 | AATATTGCAGCAGTACGCACA | G | 0.05 |
| Example 3.3.2 | | | | | 0.1 |
| Example 3.3.3 | | | | | 0.25 |
| Example 3.3.4 | | | | | 0.5 |
| Example 3.3.5 | | | | | 1 |
| Example 3.4.1 | | | AAGACTCACGTTAACAATATTG | C | 0.05 |
| Example 3.4.2 | | | | | 0.1 |
| Example 3.4.3 | | | | | 0.25 |
| Example 3.4.4 | | | | | 0.5 |
| Example 3.4.5 | | | | | 1 |

As a negative control for each experiment, the Cas 13 protein was added only without the programmable guide RNA

The experimental results are shown in Figures 12 and 13, and
wherein the detection limit was exceeded after 4 minutes of reaction in Example 3.4.5, after 8 minutes of reaction in Example 3.4.4, and after 14 minutes of reaction in Example 3.4.3, and the value of 65,000 was inserted to fill in the data points.

The experimental results demonstrate that the novel CRISPR/Cas 13 complex (1) activates the collateral cleavage function by binding to a target nucleic acid even at low concentrations, and (2) exhibits sufficient collateral cleavage activity to detect the target nucleic acid.

### Industrial Applicability

The invention disclosed in this specification relates to a novel CRISPR/Cas 13 system. The novel CRISPR/Cas 13 system can cleave a nucleic acid with a target specificity, and can also collaterally cleave any nucleic acids. The novel CRISPR/Cas 13 system can be used for cleaving non-target nucleic acids or detecting a target nucleic acid.

## Claims

1. A novel CRISPR/Cas 13 composition comprising:
a Cas 13 protein comprising a nucleic acid sequence of SEQ ID NO: 1, or a nucleic acid encoding the Cas 13 protein; and
a programmable guide RNA, or a nucleic acid encoding the programmable guide RNA,
wherein the programmable guide RNA has a structure where a direct repeat and a guide domain are linked from a 5'-end to a 3'-end direction,
wherein the direct repeat interacts with the Cas 13 protein to form a complex,
wherein the direct repeat comprises an RNA sequence of SEQ ID NO: 2, and
wherein the guide domain is capable of binding complementarily to a predetermined target nucleic acid.

2. The novel CRISPR/Cas 13 composition of claim 1,
wherein the nucleic acid encoding the Cas 13 protein is DNA or mRNA, and
wherein the nucleic acid encoding the programmable guide RNA is DNA.

3. The novel CRISPR/Cas 13 composition of claim 1 or claim 2,
wherein the novel CRISPR/Cas 13 composition comprises the Cas 13 protein and the programmable guide RNA,
wherein the Cas 13 protein and the programmable guide RNA form a protein-RNA complex.

4. The novel CRISPR/Cas 13 composition of claim 1 or claim 2,
wherein the novel CRISPR/Cas 13 composition comprises the nucleic acid encoding the Cas 13 protein and the nucleic acid encoding the programmable guide RNA.

5. The novel CRISPR/Cas 13 composition of claim 4,
wherein the nucleic acid encoding the Cas 13 protein and the nucleic acid encoding the programmable guide RNA are included in a single vector molecule.

6. The novel CRISPR/Cas 13 composition of claim 4,
wherein the nucleic acid encoding the Cas 13 protein and the nucleic acid encoding the programmable guide RNA are each included in two or more vector molecules.

7. The novel CRISPR/Cas 13 composition of claim 5 or claim 6,
wherein the vector is a viral vector, a non-viral vector, or a proper combination thereof.

8. A method for cleaving non-target RNAs using a novel CRISPR/Cas 13 system, comprising:
mixing the novel CRISPR/Cas 13 composition according to claim 3 with a sample,
wherein the sample comprises a target RNA and the non-target RNA, and
wherein the guide domain of the programmable guide RNA of the novel CRISPR/Cas 13 composition is capable of binding complementarily to the target RNA.

9. A method for detecting a target RNA using a novel CRISPR/Cas 13 system, comprising:
(a) mixing the novel CRISPR/Cas 13 composition according to claim 3, a sample, and a detecting agent,
wherein the guide domain of the programmable guide RNA of the novel CRISPR/Cas 13 composition is capable of binding complementarily to the target RNA,
wherein the detecting agent comprises a reporter, and the reporter comprises a probe nucleic acid,
wherein when the probe nucleic acid is cleaved, the reporter generates a detectable signal, and
wherein when the sample comprises the target RNA, the protein-RNA complex of the novel CRISPR/Cas 13 composition binds complementarily to the target RNA, thereby activating the collateral cleavage function of the protein-RNA complex, leading to the cleavage of the probe nucleic acid; and
(b) measuring the detectable signal in the said mixture,
wherein information on the target RNA can be obtained by measuring the detectable signal.

10. The method for detecting the target RNA of claim 9,
wherein the (b) is a process of measuring whether the detectable signal is generated; the intensity of the generated signal; the change in signal intensity over time; or any combination thereof.

11. The method for detecting the target DNA of claim 9 or claim 10,
wherein the information about the target RNA comprises whether the target RNA is present, how much of the target RNA is included in the sample, or both.

12. The method for detecting the target DNA of any one of claims 9 to 11,
wherein the detectable signal is a fluorescence signal.

13. A method for providing information on diagnosing COVID-19, comprising:
(a) mixing a novel CRISPR/Cas 13 complex, a specimen, and a detecting agent,
wherein the specimen is collected from a patient suspected of having COVID-19 infection,
wherein the novel CRISPR/Cas 13 complex comprises
a Cas 13 protein of SEQ ID NO: 1; and guide RNA,
wherein the guide RNA has a structure where a direct repeat of SEQ ID NO: 2 and a guide domain are linked sequentially from a 5' end to a 3' end,
wherein the guide domain comprises an RNA sequence selected from a group consisting of SEQ ID NOS: 37 to 51, and SEQ ID NOS: 52 to 56,
wherein the detecting agent comprises a reporter, and the reporter comprises a probe nucleic acid,
wherein when the probe nucleic acid is cleaved, the reporter generates a fluorescence signal;
(b) measuring the fluorescence signal in the mixture; and
(c) obtaining information on whether the patient who provides the specimen is infected with COVID-19, by analyzing the fluorescence signal.

14. The method for providing information on diagnosing COVID-19 of claim 13,
wherein the (b) is a process of measuring whether the fluorescence signal is generated; an intensity of the fluorescence signal generated; a change in the intensity of the fluorescence signal over time; or any combination thereof.
